(19) Europäisches Patentamt
European Patent Office
Office européen des brevets

(11) EP 4 647 427 A1

(12) **EUROPEAN PATENT APPLICATION**
published in accordance with Art. 153(4) EPC

(43) Date of publication:
**12.11.2025 Bulletin 2025/46**

(21) Application number: **24738504.0**

(22) Date of filing: **03.01.2024**

(51) International Patent Classification (IPC):
***C07D 401/06*** *(2006.01)* ***C07D 409/06*** *(2006.01)*
***C07D 405/06*** *(2006.01)* ***C07D 307/60*** *(2006.01)*
***A61K 31/365*** *(2006.01)* ***A61K 31/4439*** *(2006.01)*
***A61K 31/443*** *(2006.01)* ***A61K 31/4025*** *(2006.01)*
***A61P 25/16*** *(2006.01)* ***A61P 25/28*** *(2006.01)*
***A61P 25/08*** *(2006.01)* ***A61P 25/14*** *(2006.01)*
***A61P 25/00*** *(2006.01)*

(52) Cooperative Patent Classification (CPC):
**A61K 31/365; A61K 31/4015; A61K 31/4025; A61K 31/443; A61K 31/4439; A61P 21/00; A61P 25/00; A61P 25/08; A61P 25/14; A61P 25/16; A61P 25/28; C07D 207/38; C07D 307/60; C07D 401/06; C07D 405/06;** (Cont.)

(86) International application number:
**PCT/CN2024/070435**

(87) International publication number:
**WO 2024/146571 (11.07.2024 Gazette 2024/28)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **03.01.2023 CN 202310004796**
**22.08.2023 CN 202311060328**

(71) Applicant: **TYK Medicines, Inc.**
**Huzhou, Zhejiang 313100 (CN)**

(72) Inventors:
- **LIANG, Apeng**
  **Huzhou, Zhejiang 313100 (CN)**
- **ZHU, Jian**
  **Huzhou, Zhejiang 313100 (CN)**
- **WU, Yusheng**
  **Huzhou, Zhejiang 313100 (CN)**
- **CHEN, Shaoqing**
  **Huzhou, Zhejiang 313100 (CN)**
- **LI, Jun**
  **Huzhou, Zhejiang 313100 (CN)**
- **LIN, Jiaying**
  **Huzhou, Zhejiang 313100 (CN)**

(74) Representative: **Dompatent**
**Partnerschaft von Patent- und Rechtsanwälten mbB**
**Deichmannhaus am Dom**
**Bahnhofsvorplatz 1**
**50667 Köln (DE)**

(54) **COMPOUND FOR PREVENTING AND/OR TREATING NEURODEGENERATIVE DISEASE**

(57) The present invention relates to a compound for preventing and/or treating a neurodegenerative disease. Specifically, the compound of the present invention has a structure as shown in formula I, wherein the definitions of groups and substituents in the formula are as described in the description. Also disclosed are a preparation method for the compound and a use of the compound in preventing and/or treating a neurodegenerative disease.

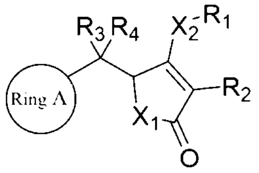

Formula I

EP 4 647 427 A1

(52) Cooperative Patent Classification (CPC): (Cont.)
**C07D 409/06**

## Description

### THCHNICAL FIFLD

The present invention relates to the field of medicine, specifically to compound for preventing and/or treating neurodegenerative disease.

### BACKGROUND ART

Alzheimer's disease, commonly known as senile dementia, is a common neurodegenerative disease characterized by memory loss and neuronal death, with the primary clinical hallmarks being senile plaques and neurofibrillary tangles. The pathological changes associated with Alzheimer's disease are highly complex, and while countries worldwide are researching effective treatments for the disease, progress has been limited. There are currently no specific treatments or medications available that can treat or reverse the progression of Alzheimer's disease. Over a decade ago, the U.S. FDA approved two classes of therapeutic compounds, totaling five compounds, including cholinesterase inhibitors and NMDA receptor antagonists. However, these medications can only temporarily alleviate symptoms and cannot halt the progression of the disease. The main pathogenesis of the disease is the deposition of β-amyloid protein in the brain tissue of the body, abnormal cerebral vascular structure causing activation of microglia and astrocytes in the brain tissue to produce inflammatory mediators, increased production of free radicals in the brain tissue accompanied by weakened antioxidant capacity, imbalance between the acetylcholine system and anti-acetylcholine system in the brain tissue, etc., leading to structural and functional damage to the brain tissue of senile dementia patients, increased content of inflammatory mediators in the hippocampus, weakened antioxidant capacity in the hippocampus, and ultimately inducing Alzheimer's disease. Pharmaceutical companies have developed various vaccines targeting the Abeta protein and phosphorylation inhibitors for multiple enzymes involved in its formation, but so far, these efforts have ended in failure. We believe that it is too late to reduce the toxicity of this protein after the patient has developed symptoms, and we should start by reducing the toxicity of this protein and developing therapeutic drugs.

In recent years, the mechanisms underlying the role of RAC1 in the nervous system have increasingly become a focal point of research. Existing literature has reported that dysregulation of physiological signals centered around the RAC1 network is associated with pathological changes in Alzheimer's disease and leads to age-dependent neurodegenerative lesions (Human Molecular Genetics, 2020, Vol. 29, No. 5). By regulating RAC1 to enhance long-term potentiation (LTP), brain learning and memory capabilities can be strengthened (PNAS, 2007, Vol. 104, No. 2). Therefore, RAC1 has emerged as a highly promising target for the treatment and prevention of neurodegenerative diseases.

### SUMMARY OF THE INVENTION

The purpose of the present invention is to provide a compound shown in Formula I, and preparation method therefor, and its use in the prevention and/or treatment of neurodegenerative diseases.

In the first aspect of the present invention, provided is a compound of formula I, or a stereoisomer, a racemate, or a pharmaceutically acceptable salt thereof,

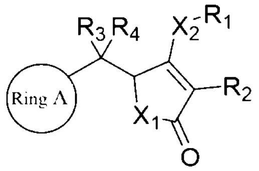

Formula I

wherein,

Ring A is selected from the group consisting of

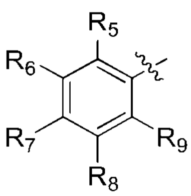

,

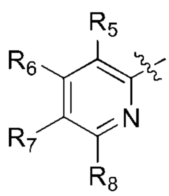

,

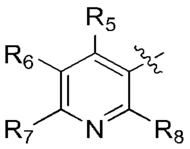 , 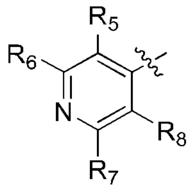 , 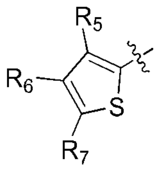 , 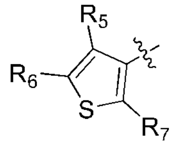 , 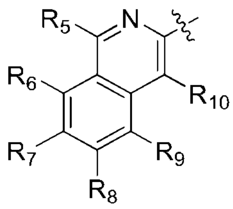 ,

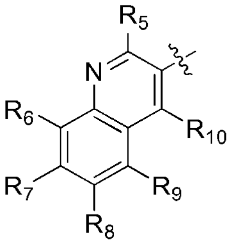 , 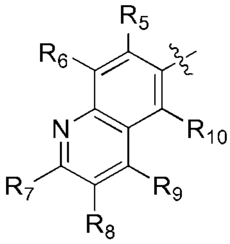 , 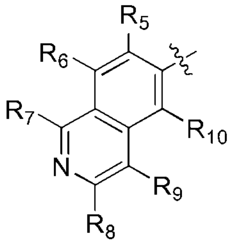 , 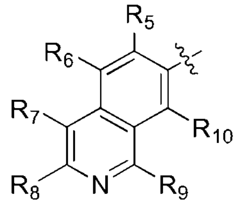 ,

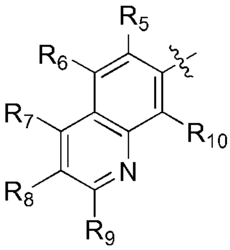 , 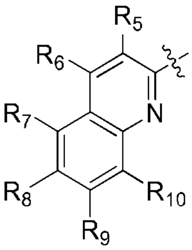 , 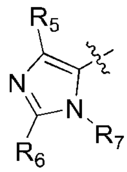 , 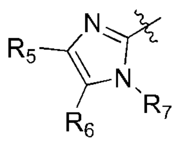 , and 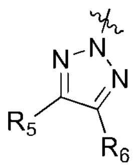 ;

$X_1$ is selected from the group consisting of O, S, NH, and NR;
$X_2$ is selected from the group consisting of O, NH, and NR;
$R_1$ is selected from the group consisting of

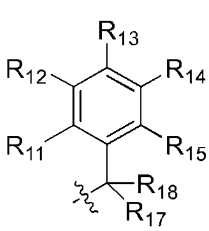 , 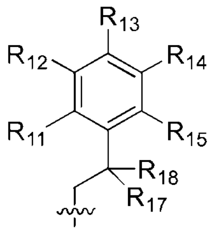 ,

and

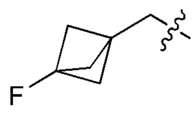 ;

$R_2$ is selected from the group consisting of H, halogen, substituted or unsubstituted C1-C6 alkyl, substituted or unsubstituted C1-C6 alkoxy, and substituted or unsubstituted C3-C6 cycloalkyl;
$R_3$ and $R_4$ are independently selected from the group consisting of H, D, halogen, substituted or unsubstituted C1-C6 alkyl, substituted or unsubstituted C1-C6 alkoxy and substituted or unsubstituted C3-C6 cycloalkyl, or $R_3$ and $R_4$ together with the carbon to which they are attached form a substituted or unsubstituted 3-7 membered cycloalkyl or a substituted or unsubstituted 3-7 membered heterocyclyl containing one or more heteroatoms selected from O, S and N;
each $R_5$, $R_6$, $R_7$, $R_8$, $R_9$, and $R_{10}$ is independently selected from the group consisting of H, D, halogen, trifluoromethyl, cyano, hydroxy, amino, substituted or unsubstituted C1-C6 alkyl, substituted or unsubstituted C1-C6 alkoxy, substituted or unsubstituted C1-C6 alkyl-$NR_{16}$-, substituted or unsubstituted C3-C6 cycloalkyl, substituted or unsubstituted C3-C6 cycloalkoxy and substituted or unsubstituted C3-C6 cycloalkyl-$NR_{16}$-;

each $R_{11}$, $R_{12}$, $R_{13}$, $R_{14}$, and $R_{15}$ is independently selected from the group consisting of H, D, halogen, trifluoromethyl, cyano, hydroxy, amino, substituted or unsubstituted C1-C6 alkyl, substituted or unsubstituted C1-C6 alkoxy, substituted or unsubstituted C1-C6 alkyl-$NR_{16}$-, substituted or unsubstituted C3-C6 cycloalkyl, substituted or unsubstituted C3-C6 cycloalkoxy and substituted or unsubstituted C3-C6 cycloalkyl-$NR_{16}$-;
each $R_{17}$ and $R_{18}$ is independently selected from the group consisting of H, D, C1-C6 alkyl, C1-C6 alkoxy, C3-C6 cycloalkyl and C3-C6 cycloalkoxy; or $R_{17}$ and $R_{18}$ together with the carbon to which they are attached form a substituted or unsubstituted 3-7 membered cycloalkyl or a substituted or unsubstituted 3-7 membered heterocyclyl containing one or more heteroatoms selected from O, S and N;
each R is independently a substituted or unsubstituted C1-C6 alkyl;
the "substituted" each independently refers to be substituted by one or more substituents selected from the group consisting of D, halogen, trifluoromethyl, cyano, hydroxy, amino, C1-C6 alkyl, C1-C6 alkoxy, C1-C6 alkyl-$NR_{16}$-, C3-C6 cycloalkyl, C3-C6 cycloalkoxy and C3-C6 cycloalkyl-$NR_{16}$-;
each $R_{16}$ is independently selected from the group consisting of H and C1-C6 alkyl;
the premise is that when $X_1$ is NH, $X_2$ is O, and $R_3$, $R_4$, $R_{17}$ and $R_{18}$ are H, ring A is not

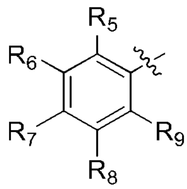
.

In another preferred embodiment, the compound has a structure of formula II:

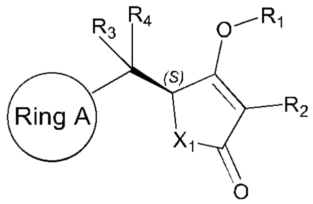
Formula II

wherein, ring A, $X_1$, $R_1$, $R_2$, $R_3$, and $R_4$ are as defined above.
In another preferred embodiment, ring A is selected from the group consisting of

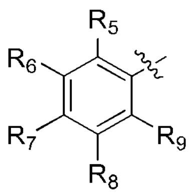
,
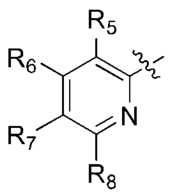
,
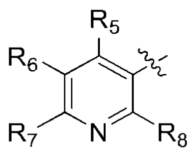
,
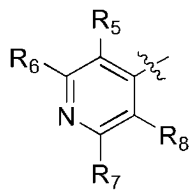
,
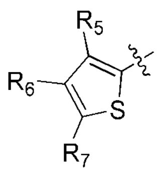
, and
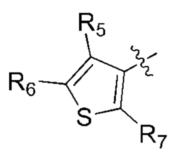
;

$R_5$, $R_6$, $R_7$, $R_8$, and $R_9$ are as defined above.
In another preferred embodiment, $X_1$ is selected from the group consisting of O, S, NH, and NR;
R is as defined above.
In another preferred embodiment, $X_2$ is selected from the group consisting of O, NH, and NR;
R as defined above.
In another preferred embodiment, $R_1$ is selected from the group consisting of

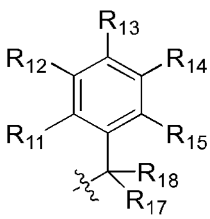 , 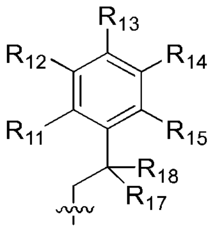 , and 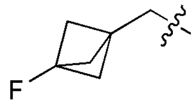 ;

each $R_{11}$, $R_{12}$, $R_{13}$, $R_{14}$, $R_{15}$, $R_{17}$, and $R_{18}$ is as defined above.

In another preferred embodiment, $R_2$ is selected from the group consisting of H, halogen, substituted or unsubstituted C1-C6 alkyl, substituted or unsubstituted C1-C6 alkoxy and substituted or unsubstituted C3-C6 cycloalkyl;

the "substituted" each independently refers to be substituted by one or more substituents selected from the group consisting of D, halogen, trifluoromethyl, cyano, hydroxy, amino, C1-C6 alkyl, C1-C6 alkoxy, C1-C6 alkyl-$NR_{16}$-, C3-C6 cycloalkyl, C3-C6 cycloalkoxy and C3-C6 cycloalkyl-$NR_{16}$-;
each $R_{16}$ is independently selected from the group consisting of H and C1-C6 alkyl.

In another preferred embodiment, $R_3$ and $R_4$ are independently selected from the group consisting of H, D, halogen, substituted or unsubstituted C1-C6 alkyl, substituted or unsubstituted C1-C6 alkoxy and substituted or unsubstituted C3-C6 cycloalkyl, or $R_3$ and $R_4$ together with the carbon to which they are attached form a substituted or unsubstituted 3-7 membered cycloalkyl or a substituted or unsubstituted 3-7 membered heterocyclyl containing one or more heteroatoms selected from O, S and N.

In another preferred embodiment, each $R_5$, $R_6$, $R_7$, $R_8$, $R_9$, and $R_{10}$ is independently selected from the group consisting of H, D, halogen, trifluoromethyl, cyano, hydroxy, amino, substituted or unsubstituted C1-C6 alkyl, substituted or unsubstituted C1-C6 alkoxy, substituted or unsubstituted C1-C6 alkyl-$NR_{16}$-, substituted or unsubstituted C3-C6 cycloalkyl, substituted or unsubstituted C3-C6 cycloalkoxy and substituted or unsubstituted C3-C6 cycloalkyl-$NR_{16}$-;
each $R_{16}$ is independently selected from the group consisting of H and C1-C6 alkyl.

In another preferred embodiment, each $R_5$, $R_6$, $R_7$, $R_8$, $R_9$, and $R_{10}$ is independently selected from the group consisting of H, D, halogen, cyano, hydroxy, amino, substituted or unsubstituted C1-C6 alkyl, substituted or unsubstituted C1-C6 alkoxy, substituted or unsubstituted C1-C6 alkyl-$NR_{16}$-, substituted or unsubstituted C3-C6 cycloalkyl, substituted or unsubstituted C3-C6 cycloalkoxy and substituted or unsubstituted C3-C6 cycloalkyl-$NR_{16}$-;
each $R_{16}$ is independently selected from the group consisting of H and C1-C6 alkyl.

In another preferred embodiment, each $R_{11}$, $R_{12}$, $R_{13}$, $R_{14}$, and $R_{15}$ is independently selected from the group consisting of H, D, halogen, trifluoromethyl, cyano, hydroxy, amino, substituted or unsubstituted C1-C6 alkyl, substituted or unsubstituted C1-C6 alkoxy, substituted or unsubstituted C1-C6 alkyl-$NR_{16}$-, substituted or unsubstituted C3-C6 cycloalkyl, substituted or unsubstituted C3-C6 cycloalkoxy and substituted or unsubstituted C3-C6 cycloalkyl-$NR_{16}$-;
each $R_{16}$ is independently selected from the group consisting of H and C1-C6 alkyl.

In another preferred embodiment, each $R_{17}$ and $R_{18}$ is independently selected from the group consisting of H, D, C1-C6 alkyl, C1-C6 alkoxy, C3-C6 cycloalkyl and C3-C6 cycloalkoxy; or $R_{17}$ and $R_{18}$ together with the carbon to which they are attached form a substituted or unsubstituted 3-7 membered cycloalkyl or a substituted or unsubstituted 3-7 membered heterocyclyl containing one or more heteroatoms selected from O, S and N.

In another preferred embodiment, $R_{17}$ is selected from the group consisting of H and D.

In another preferred embodiment, $R_{18}$ is C1-C6 alkyl.

In another preferred embodiment, when ring A is

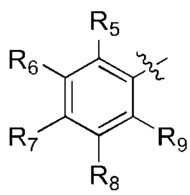 ,

$R_3$, $R_4$, $R_{17}$, and $R_{18}$ are not simultaneously H.

In another preferred embodiment, when ring A is

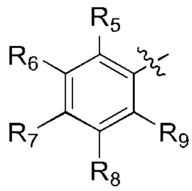
,

$R_5$, $R_6$, $R_7$, $R_8$, and $R_9$ are as defined above.
$R_3$ and $R_4$ are independently selected from the group consisting of H and C1-C6 alkyl;
$R_{17}$ and $R_{18}$ are independently selected from the group consisting of H, D, and C1-C6 alkyl;
and, $R_3$, $R_4$, $R_{17}$, and $R_{18}$ are not simultaneously H.

In another preferred embodiment, when ring A is

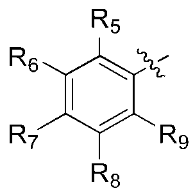
,

$R_5$, $R_6$, $R_7$, $R_8$, and $R_9$ are as defined above;
$R_3$ and $R_4$ are H;
$R_{17}$ and $R_{18}$ are independently selected from the group consisting of H, D, and C1-C6 alkyl;
and, $R_{17}$ and $R_{18}$ are not simultaneously H.

In another preferred embodiment, when ring A is

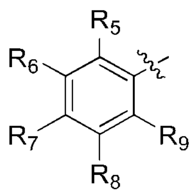
,

$R_5$, $R_6$, $R_7$, $R_8$, and $R_9$ are as defined above;
$R_3$ is H;
$R_4$ is C1-C6 alkyl;
$R_{17}$ and $R_{18}$ are H.

In another preferred embodiment, when ring A is selected from the group consisting of

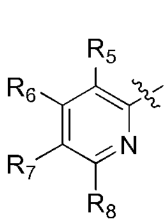 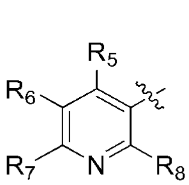 , 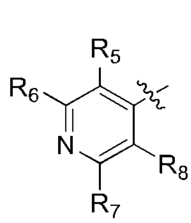 , 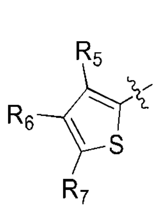 ,

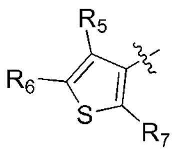, 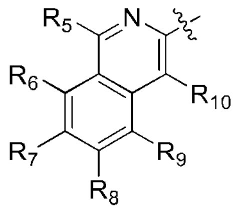, 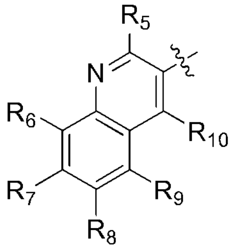, 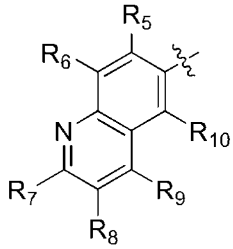,

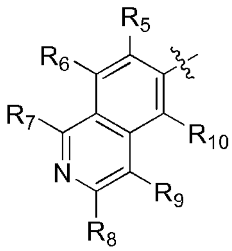, 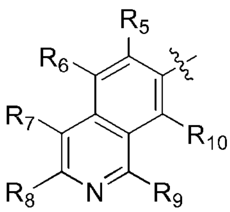, 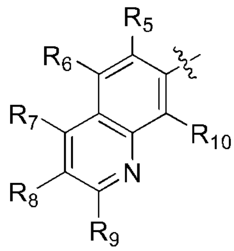, 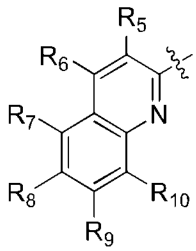, 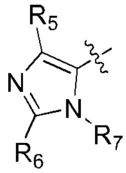,

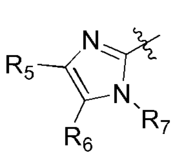, and 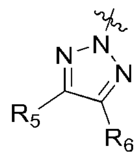,

$R_5$, $R_6$, $R_7$, $R_8$, $R_9$ and $R_{10}$ are as defined above.

$R_3$ and $R_4$ are independently selected from the group consisting of H, D, halogen, substituted or unsubstituted C1-C6 alkyl, substituted or unsubstituted C1-C6 alkoxy and substituted or unsubstituted C3-C6 cycloalkyl, or $R_3$ and $R_4$ together with the carbon to which they are attached form a substituted or unsubstituted 3-7 membered cycloalkyl or a substituted or unsubstituted 3-7 membered heterocyclyl containing one or more heteroatoms selected from O, S and N;

each $R_{17}$ and $R_{18}$ is independently selected from the group consisting of H, D, C1-C6 alkyl, C1-C6 alkoxy, C3-C6 cycloalkyl and C3-C6 cycloalkoxy; or $R_{17}$ and $R_{18}$ together with the carbon to which they are attached form a substituted or unsubstituted 3-7 membered cycloalkyl or a substituted or unsubstituted 3-7 membered heterocyclyl containing one or more heteroatoms selected from O, S and N;

the "substituted" each independently refers to be substituted by one or more substituents selected from the group consisting of D, halogen, trifluoromethyl, cyano, hydroxy, amino, C1-C6 alkyl, C1-C6 alkoxy, C1-C6 alkyl-$NR_{16}$-, C3-C6 cycloalkyl, C3-C6 cycloalkoxy and C3-C6 cycloalkyl-$NR_{16}$-;

each $R_{16}$ is independently selected from the group consisting of H and C1-C6 alkyl.

In another preferred embodiment, when ring A is

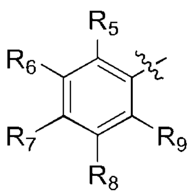,

$R_5$, $R_6$, $R_7$, and $R_8$ are as defined above;

$R_3$ and $R_4$ are independently selected from the group consisting of H, D, halogen, substituted or unsubstituted C1-C6 alkyl, substituted or unsubstituted C1-C6 alkoxy and substituted or unsubstituted C3-C6 cycloalkyl;

each $R_{17}$ and $R_{18}$ is independently selected from the group consisting of H, D, C1-C6 alkyl, C1-C6 alkoxy, C3-C6 cycloalkyl and C3-C6 cycloalkoxy;

the "substituted" each independently refers to be substituted by one or more substituents selected from the group consisting of D, halogen, trifluoromethyl, cyano, hydroxy, amino, C1-C6 alkyl, C1-C6 alkoxy, C1-C6 alkyl-$NR_{16}$-, C3-

C6 cycloalkyl, C3-C6 cycloalkoxy, C3-C6 cycloalkyl-$NR_{16}$-;
each $R_{16}$ is independently selected from the group consisting of H and C1-C6 alkyl.

In another preferred embodiment, when ring A is

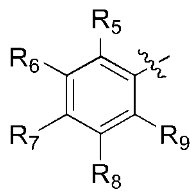

,

$R_5$, $R_6$, $R_7$, and $R_8$ are as defined above;
$R_3$ and $R_4$ are independently selected from the group consisting of H, D, halogen, C1-C6 alkyl, C1-C6 alkoxy and C3-C6 cycloalkyl;
each $R_{17}$ and $R_{18}$ is independently selected from the group consisting of H, D, C1-C6 alkyl, C1-C6 alkoxy, C3-C6 cycloalkyl and C3-C6 cycloalkoxy.

In another preferred embodiment, when ring A is

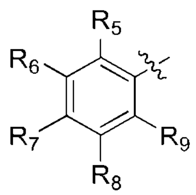

,

$X_1$ is selected from the group consisting of NH and NR;
$X_2$ is O;
$R_1$ is

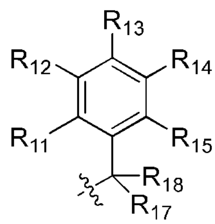

;

$R_2$ is H;
$R_3$ and $R_4$ are selected from the group consisting of H and C1-C6 alkyl;
each $R_5$, $R_6$, $R_7$, $R_8$, and $R_9$ is H;
each $R_{11}$, $R_{12}$, $R_{13}$, $R_{14}$, and $R_{15}$ is independently selected from the group consisting of H and halogen;
each $R_{17}$ and $R_{18}$ is independently selected from the group consisting of H, D and C1-C6 alkyl;
each R is independently an unsubstituted C1-C6 alkyl.

In another preferred embodiment, ring A is

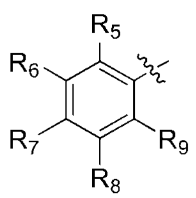

;

$X_1$ is NH;
$X_2$ is O;
$R_1$ is

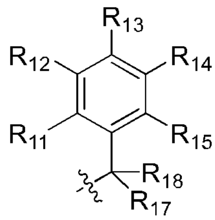

;

$R_2$ is H;
$R_3$ and $R_4$ are independently selected from the group consisting of H and C1-C6 alkyl;
each $R_5$, $R_6$, $R_7$, $R_8$, and $R_9$ is H;
each $R_{11}$, $R_{12}$, $R_{13}$, $R_{14}$, and $R_{15}$ is H;
$R_{13}$ is a halogen, preferably F;
$R_{17}$ is selected from the group consisting of H and D;
$R_{18}$ is selected from the group consisting of H, D, and C1-C6 alkyl.

In another preferred embodiment, ring A is

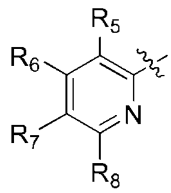

;

$X_1$ is selected from the group consisting of NH and NR;
$X_2$ is O;
$R_1$ is

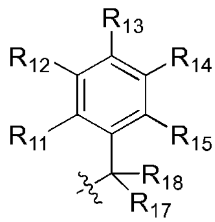

;

$R_2$ is H;
$R_3$ and $R_4$ are H;
each $R_5$, $R_6$, $R_7$, $R_8$, and $R_9$ is H and halogen;
each $R_{11}$, $R_{12}$, $R_{13}$, $R_{14}$, and $R_{15}$ is independently selected from the group consisting of H and halogen;
each $R_{17}$ and $R_{18}$ is independently selected from the group consisting of H and C1-C6 alkyl;
each R is independently an unsubstituted C1-C6 alkyl.

In another preferred embodiment, ring A is

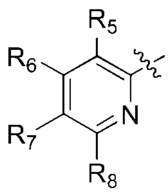

;

$X_1$ is NH;
$X_2$ is O;
$R_1$ is

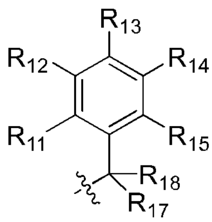

;

$R_2$ is H;
$R_3$ and $R_4$ are H;
$R_5$ is selected from the group consisting of H and halogen;
each $R_6$, $R_7$, and $R_8$ is H;
each $R_{11}$, $R_{12}$, $R_{14}$, and $R_{15}$ is H;
$R_{13}$ is halogen, preferably F;
$R_{17}$ is H;
$R_{18}$ is selected from the group consisting of H and C1-C6 alkyl.

In another preferred embodiment, $R_2$ is selected from the group consisting of H, halogen, C1-C6 alkyl, halogenated C1-C6 alkyl, C1-C6 alkoxy and C3-C6 cycloalkyl;
$R_3$ and $R_4$ are independently selected from the group consisting of H, D, halogen, C1-C6 alkyl, C1-C6 alkoxy and C3-C6 cycloalkyl.

In another preferred embodiment, ring A is

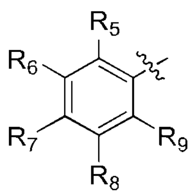

and

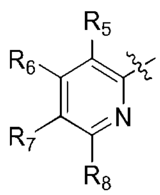

;

$R_1$ is

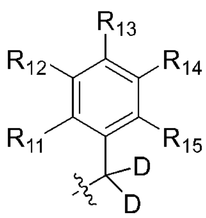

,

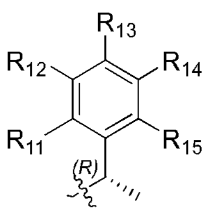

,

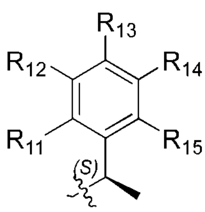

,

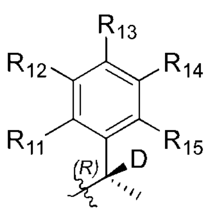

,

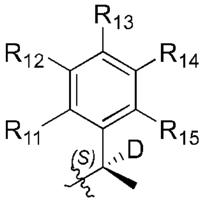
;

each $R_5$, $R_6$, $R_7$, $R_8$, $R_9$, and $R_{10}$ is independently selected from the group consisting of H, halogen, trifluoromethyl, cyano, hydroxy, amino, C1-C6 alkyl, C1-C6 alkoxy, C3-C6 cycloalkyl and C3-C6 cycloalkoxy;
each $R_{11}$, $R_{12}$, $R_{13}$, $R_{14}$, and $R_{15}$ is independently selected from the group consisting of H, halogen, trifluoromethyl, cyano, hydroxy, amino, C1-C6 alkyl, C1-C6 alkoxy, C3-C6 cycloalkyl and C3-C6 cycloalkoxy.

In another preferred embodiment, the compound is selected from the group consisting of:

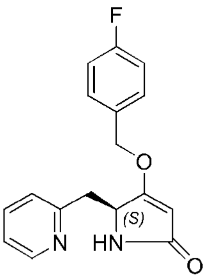
,
T-01

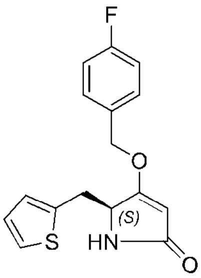
,
T-02

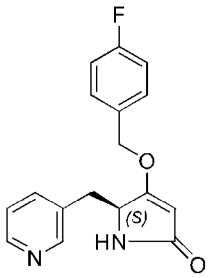
,
T-03

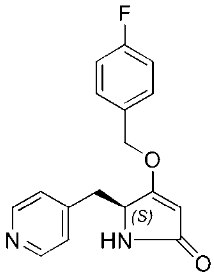
,
T-04

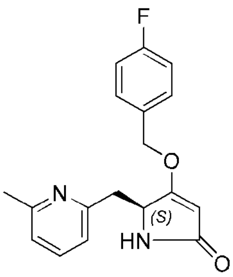
,
T-05

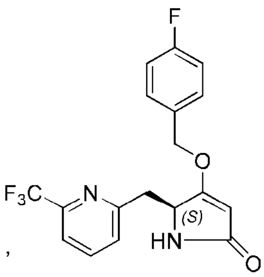
,
T-06

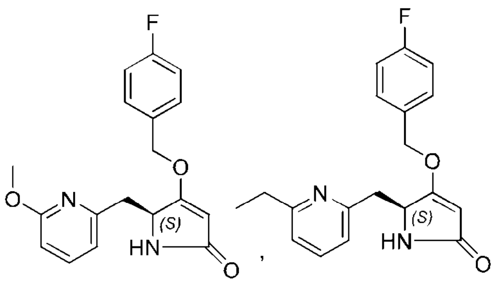
T-07
,
T-08
,

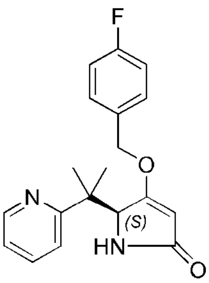
,
T-09

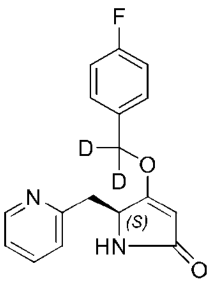
,
T-10

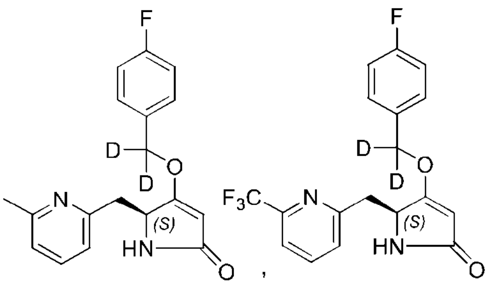
T-11
,
T-12
,

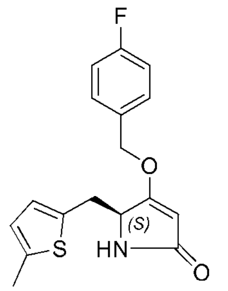 ,

T-13

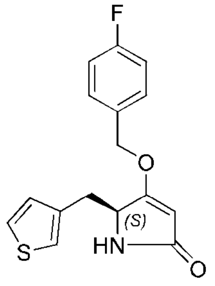 ,

T-14

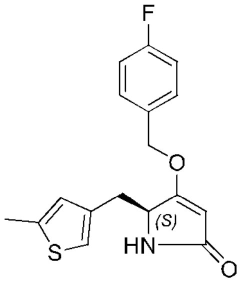 ,

T-15

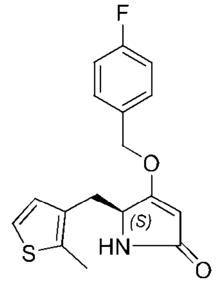 ,

T-16

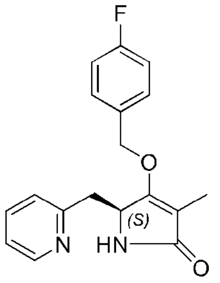 ,

T-17

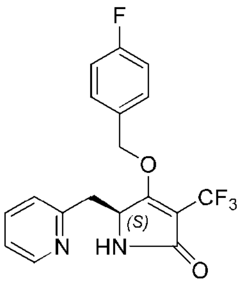 ,

T-18

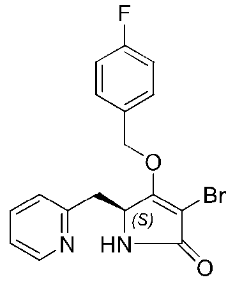 ,

T-19

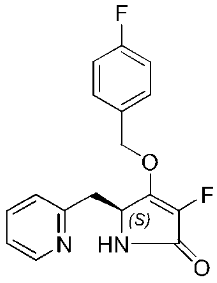 ,

T-20

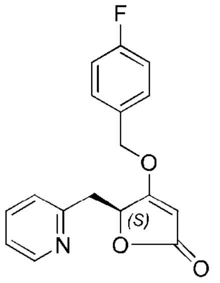 ,

T-21

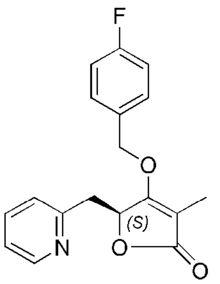 ,

T-22

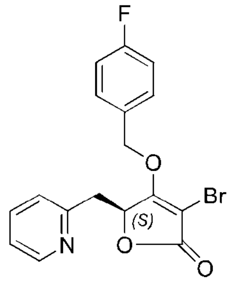 ,

T-23

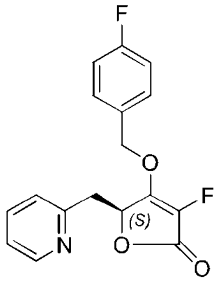 ,

T-24

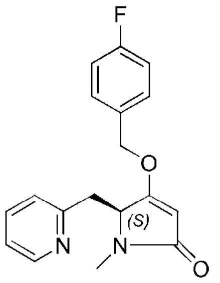 ,

T-25

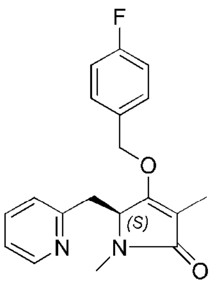 ,

T-26

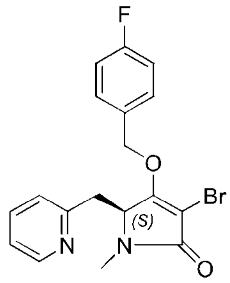 ,

T-27

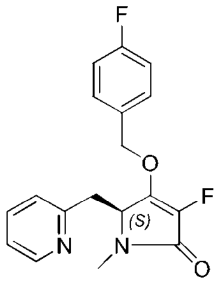 ,

T-28

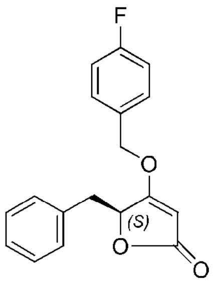 ,

T-29

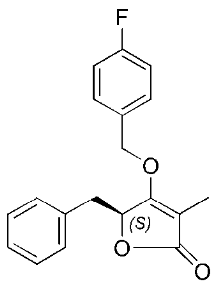 ,

T-30

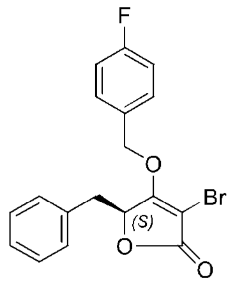 ,

T-31

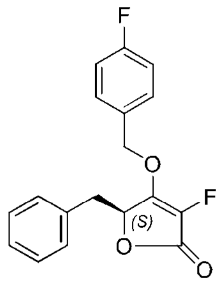 ,

T-32

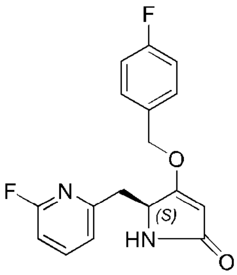 ,

T-33

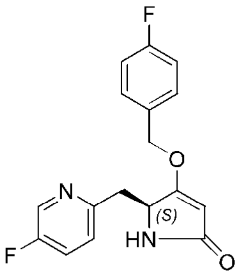 ,

T-34

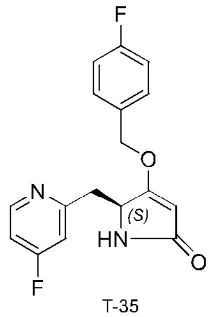 ,

T-35

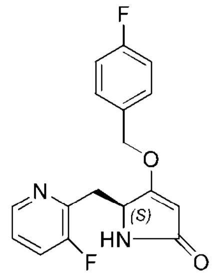 ,

T-36

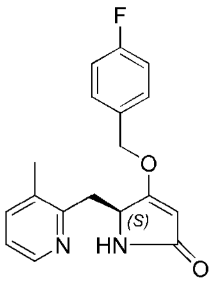 ,

T-37

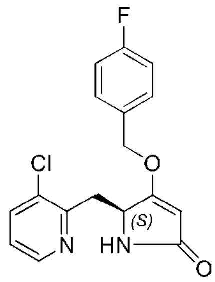 ,

T-38

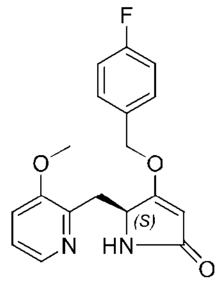 ,

T-39

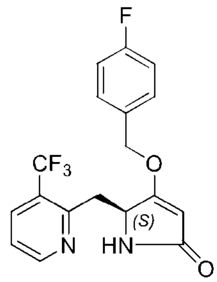 ,

T-40

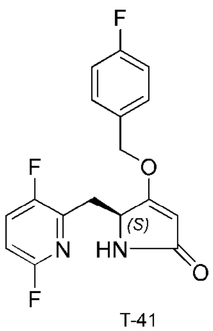 ,

T-41

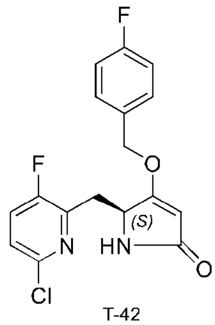 ,

T-42

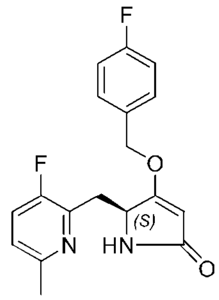 ,

T-43

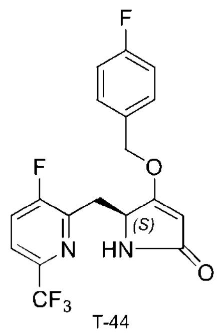 ,

T-44

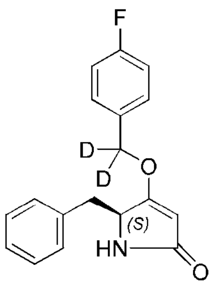 ,

T-45

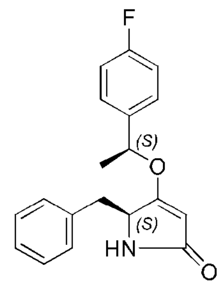 ,

T-46

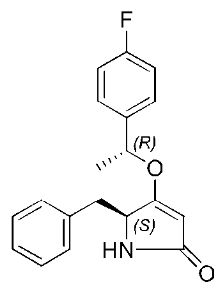 ,

T-47

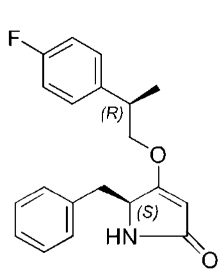 ,

T-48

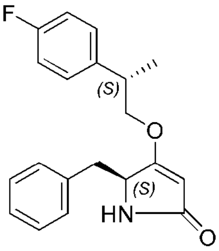 ,
T-49

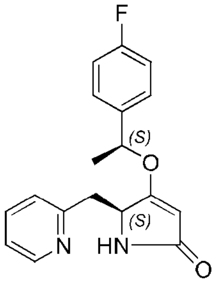 ,
T-50

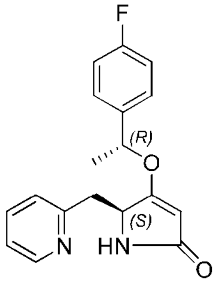 ,
T-51

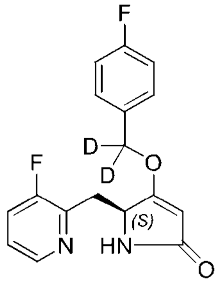 ,
T-52

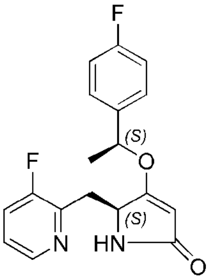 ,
T-53

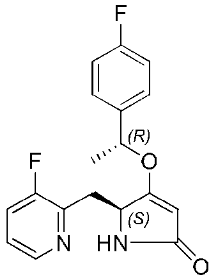 ,
T-54

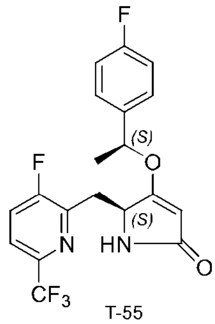 ,
T-55

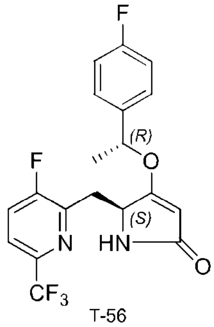 ,
T-56

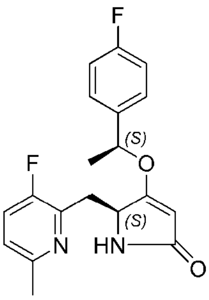 ,
T-57

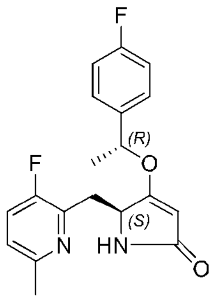 ,
T-58

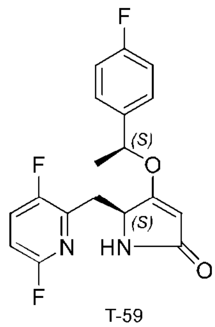 ,
T-59

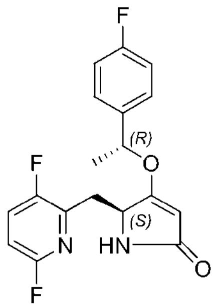 ,
T-60

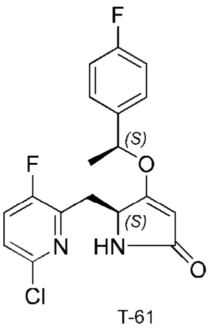 ,
T-61

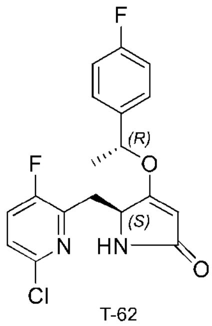 ,
T-62

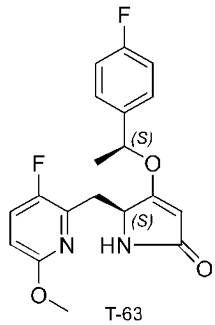 ,
T-63

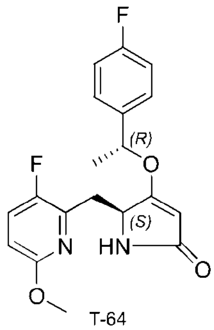 ,
T-64

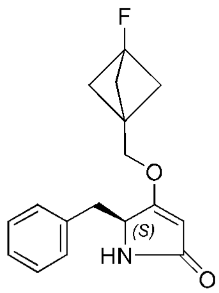,

T-65

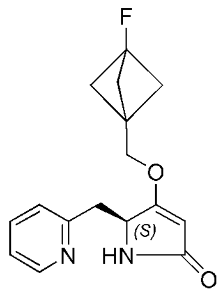,

T-66

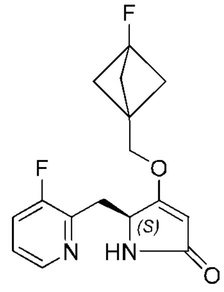,

T-67

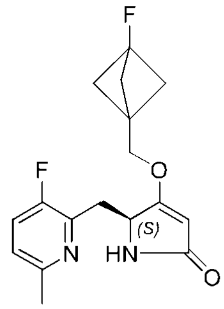,

T-68

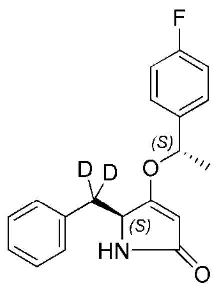,

T-69

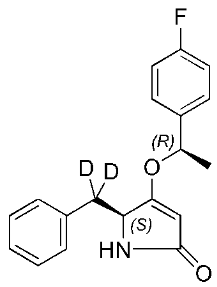,

T-70

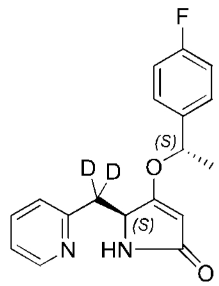,

T-71

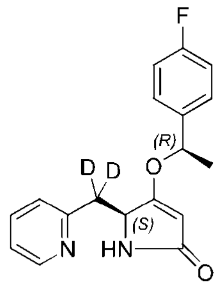,

T-72

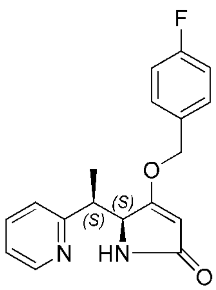,

T-73

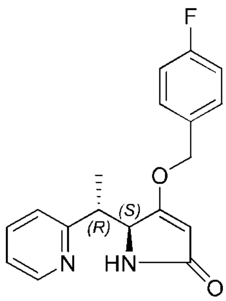,

T-74

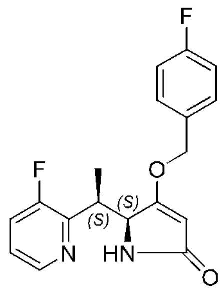,

T-75

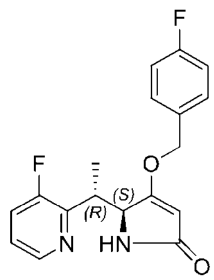,

T-76

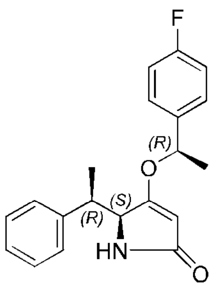,

T-77

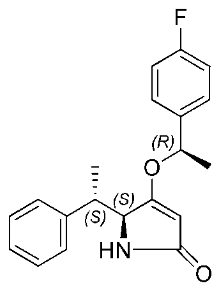,

T-78

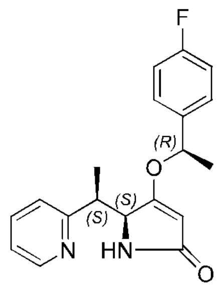,

T-79

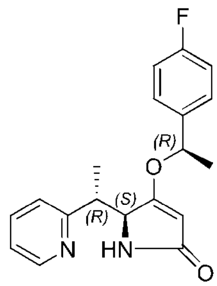,

T-80

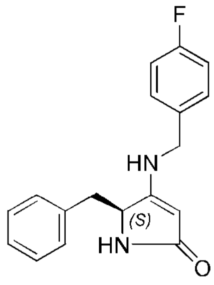,
T-81

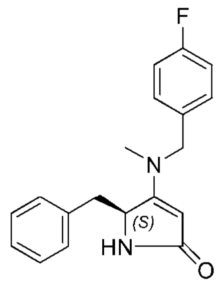,
T-82

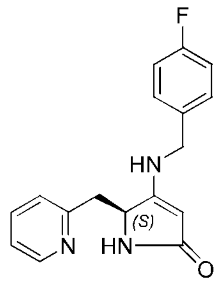,
T-83

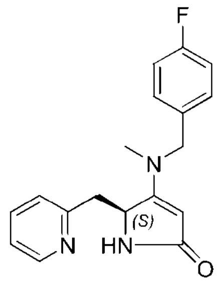,
T-84

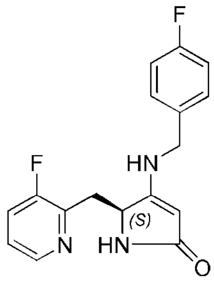,
T-85

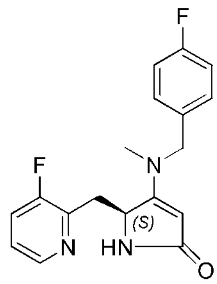,
T-86

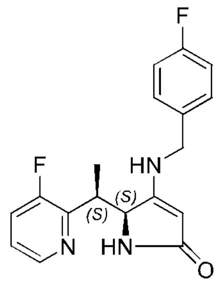,
T-87

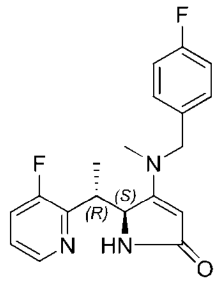,
T-88

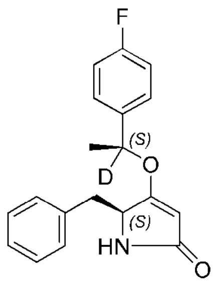,
T-89

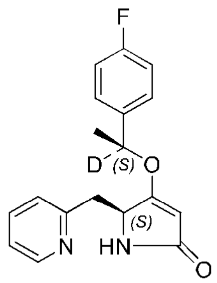,
T-90

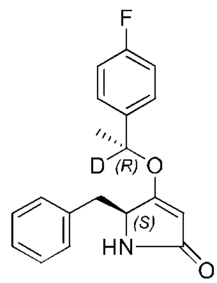,
T-91

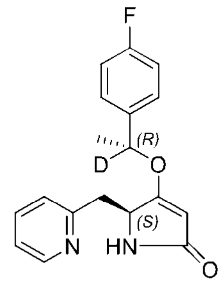,
T-92

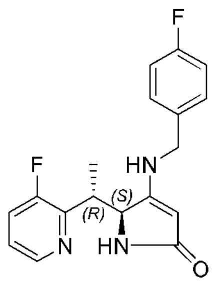,
T-93

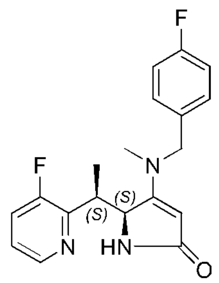,
T-94

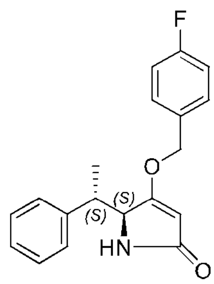,
T-95

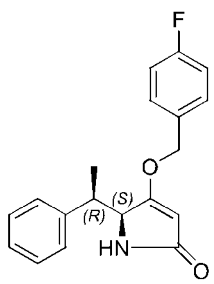,
T-96

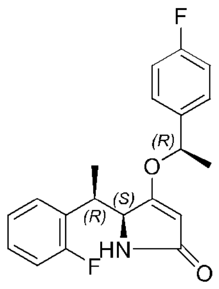 ,

T-97

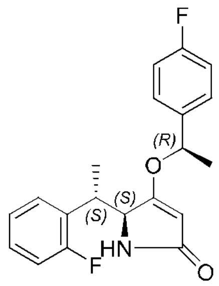 ,

T-98

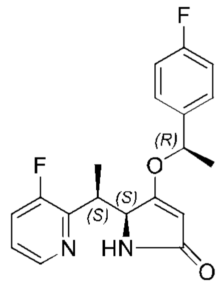 ,

T-99

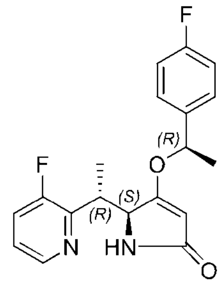 ,

T-100

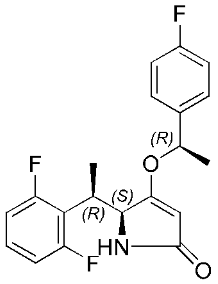 ,

T-101

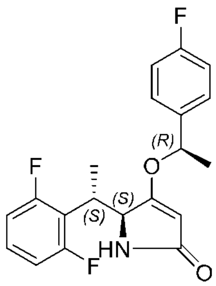 ,

T-102

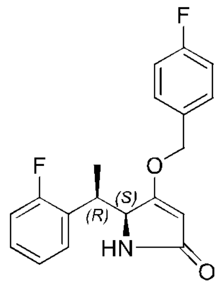 ,

T-103

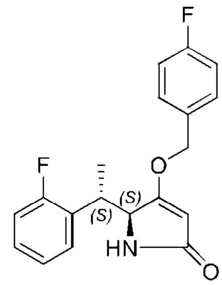 ,

T-104

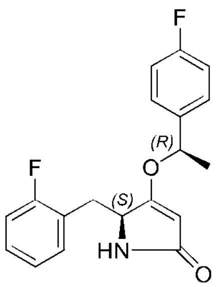 ,

T-105

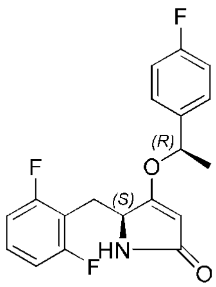 ,

T-106

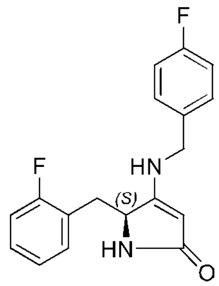 ,

T-107

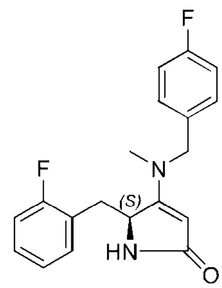 ,

T-108

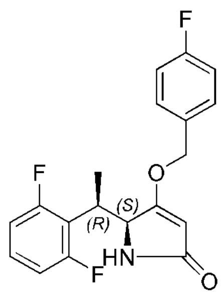 ,

T-109

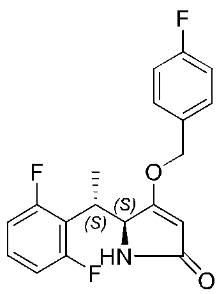 ,

T-110

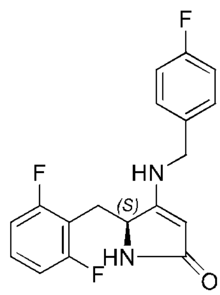 ,

T-111

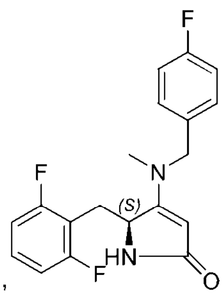 ,

T-112

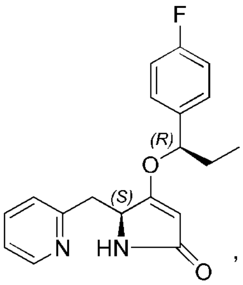 ,
T-113

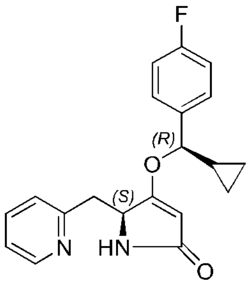 ,
T-114

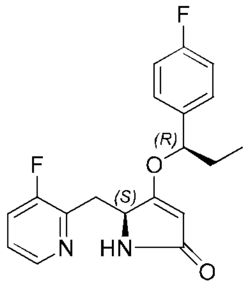 ,
T-115

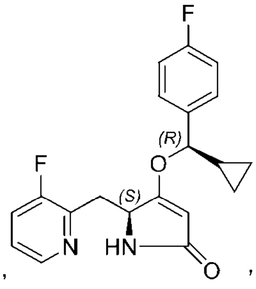 ,
T-116

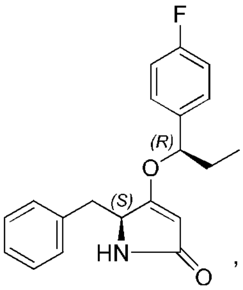 ,
T-117

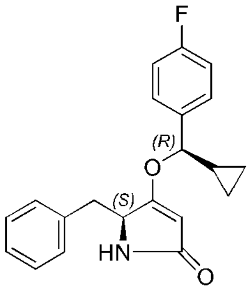 ,
T-118

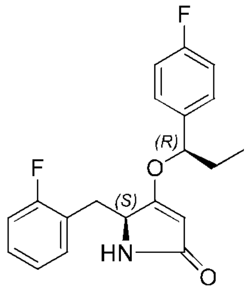 ,
T-119

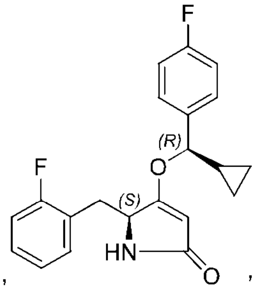 ,
T-120

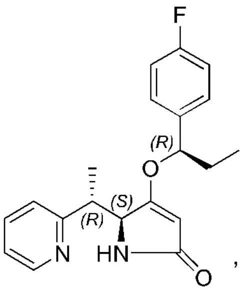 ,
T-121

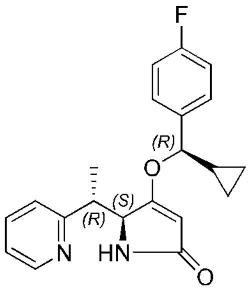 ,
T-122

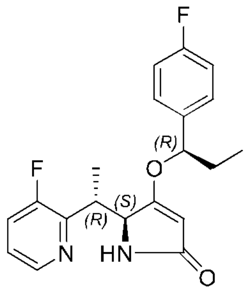 ,
T-123

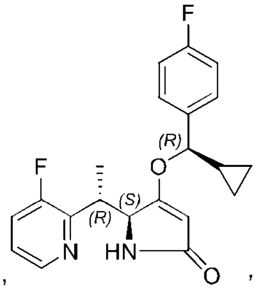 ,
T-124

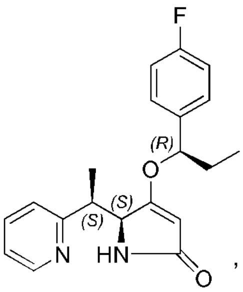 ,
T-125

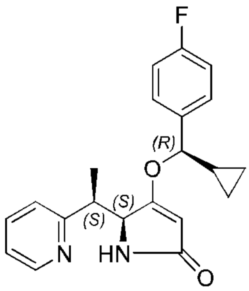 ,
T-126

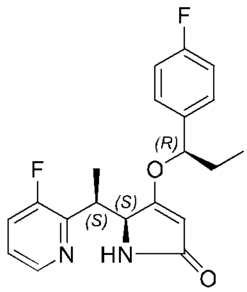 ,
T-127

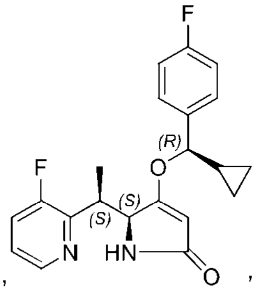 ,
T-128

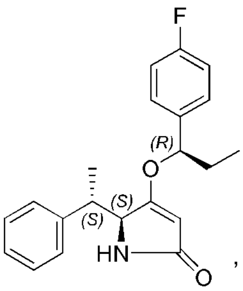
,

T-129

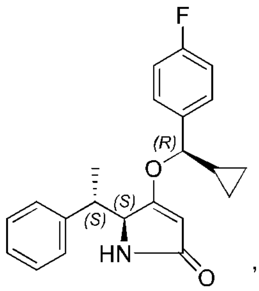
,

T-130

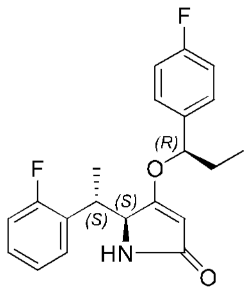
,

T-131

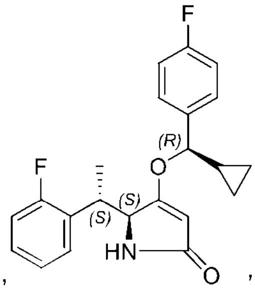
,

T-132

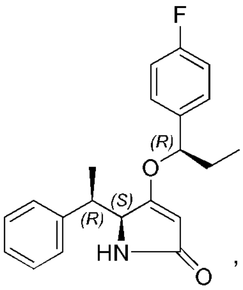
,

T-133

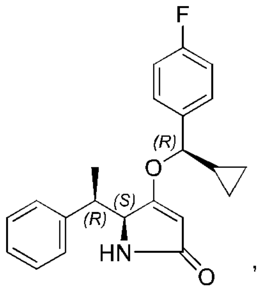
,

T-134

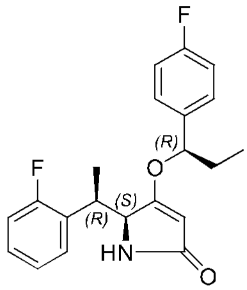
,

T-135

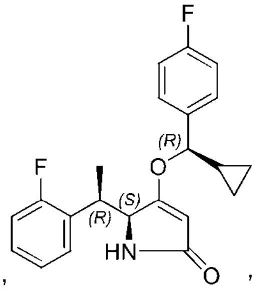
,

T-136

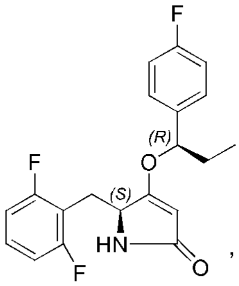
,

T-137

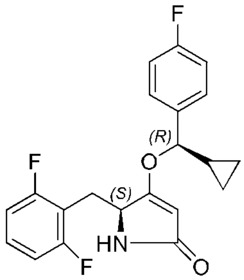
,

T-138

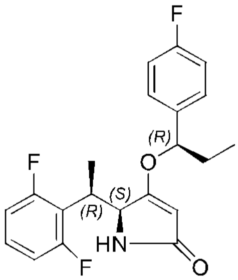
,

T-139

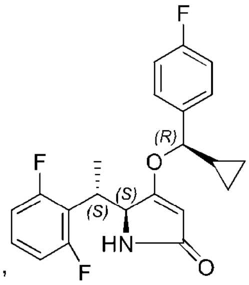

T-140

.

In another preferred embodiment, the pharmaceutically acceptable salt is an inorganic acid salt or an organic acid salt;

the inorganic acid salts are selected from the group consisting of hydrochlorate, hydrobromate, hydroiodate, sulfate, bisulfate, nitrate, phosphate, and acid phosphate;
the organic acid salts are selected from the group consisting of formate, acetate, trifluoroacetate, propionate, pyruvate, hydroxyacetate, oxalate, malonate, fumarate, maleate, lactate, malate, citrate, tartrate, methane sulfonate, ethane sulfonate, benzenesulfonate salts, p-toluenesulfonate salts, salicylate salts, picrate salts, glutamate salts, ascorbate salts, camphorate salts, and camphorsulfonate salts.

In the second aspect of the present invention, provided is a pharmaceutical composition comprising a pharmaceutically acceptable carrier and one or more safe and effective amounts of the compound of the first aspect of the present invention.
In the third aspect of the present invention, provided is a use of the compound of the first aspect of the present invention in the preparation of a medicament, and the medicament is used for the prevention and/or treatment of neurodegenerative diseases.
In another preferred embodiment, the neurodegenerative disease is selected from the group consisting of Alzheimer's disease, epilepsy, Parkinson's disease, Huntington's disease, amyotrophic lateral sclerosis, and spinocerebellar ataxia.
In the fourth aspect of the present invention, provided is a use of the compound of the first aspect of the present

invention in the preparation of a medicament, the medicament is used for the prevention and/or treatment of RAC1-related diseases.

In another preferred embodiment, the RAC1-related disease is a neurodegenerative disease.

It should be understood that in the present invention, any of the technical features specifically described above and below (such as in the Examples) can be combined with each other, so as to constitute new or preferred technical solutions. Due to space limitations, it will not redundantly be described one by one herein.

## DETAILED DESCRIPTION OF THE INVENTION

After long-term and in-depth research, the inventors unexpectedly prepared a compound with excellent pharmacokinetic properties that can effectively prevent and/or treat neurodegenerative diseases. On above basis, the inventors have completed the present invention.

## TERMS

In the present invention, unless otherwise indicated, the terms used have the ordinary meaning known to the skilled person in the art.

In the present invention, the term "halogen" refers to F, Cl, Br or I.

In the present invention, the term "C1-C6 alkyl" refers to straight-chain or branched alkyl comprising 1-6 carbon atoms, for example a methyl, ethyl, propyl, iso-propyl, butyl, iso-butyl, tert-butyl, neo-pentyl, tert-pentyl, or the like.

In the present invention, the term "C2-C6 alkenyl" refers to a straight or branched alkenyl with 2-6 carbon atoms containing a double bond, and non-limitingly includes vinyl, propenyl, butenyl, iso-butenyl, pentenyl, hexenyl and the like.

In the present invention, the term "C2-C6 alkynyl" refers to a straight or branched alkynyl with 2-6 carbon atoms containing a triple bond, and non-limitingly includes ethynyl, propynyl, butynyl, iso-butynyl, pentynyl and hexynyl, etc.

In the present invention, the term "C3-C8 cycloalkyl" refers to a cyclic alkyl group with 3-8 carbon atoms on the ring, and non-limitingly includes cyclopropyl, cyclobutyl, cyclopentyl, cyclohexyl, cycloheptyl, cyclooctyl, and the like. The term "C3-C6 cycloalkyl" has a similar meaning.

In the present invention, the term "C1-C6 alkoxy" refers to straight-chain or branched alkoxy with 1-6 carbon atoms, and non-limitingly includes methoxy, ethoxy, propoxy, iso-propoxy and butoxy. Preferably C1-C4 alkoxy.

In the present invention, the term "heterocyclyl" refers to a heterocyclic group with 4-8 ring atoms containing 1, 2 or 3 heteroatoms selected from N, O and S, including but not limited to the following groups:

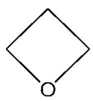 , 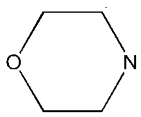 ,

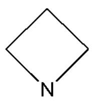 , 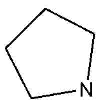 , and 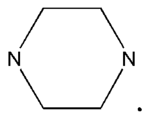 .

In the present invention, the terms "aryl ring" or "aryl" have the same meaning, preferably "C6-C10 aryl". The term "C6-C10 aryl" refers to an aromatic ring with 6-10 carbon atoms without heteroatoms on the ring, such as phenyl, naphthyl, etc.

In the present invention, the term "aromatic heterocycle" or "heteroaryl" has the same meaning and refers to a heteroaromatic group containing one to more heteroatoms. For example, "C3-C10 heteroaryl" refers to an aromatic heterocyclic ring with 3 to 10 carbon atoms containing 1 to 4 heteroatoms selected from oxygen, sulfur, and nitrogen. Non-limiting examples are furanyl, thienyl, pyridinyl, pyrazolyl, pyrrolyl, N-alkylpyrrolyl, pyrimidinyl, pyrazinyl, imidazolyl, tetrazolyl, etc. The heteroaryl ring may be fused on an aryl, heterocyclyl or cycloalkyl ring, wherein the ring attached to the parent structure is a heteroaryl ring. The heteroaryl may be optionally substituted or unsubstituted.

In the present invention, the term "halogenated" means substituted with a halogen.

In the present invention, the term "substituted" refers to one or more hydrogen atoms on a particular moiety is substituted by a particular substituent. The particular substituent is a substituent described above accordingly, or a substituent in the embodiments. Unless indicated specifically, a substituted group may have a substituent selected from a particular group at any substitutable site of the group, and substituents can be identical or different at each site. It should be understood by the person skilled in the art that the combinations of substituents expected in the present invention are ones that are stable or chemically realizable. For example, substituents are (but not limited to) halogen, hydroxyl, carboxy

(-COOH), C1-C6 alkyl, C2-C6 alkenyl, C2-C6 alkynyl, C3-C8 cycloalkyl, 3-12 membered heterocyclyl, aryl, heteroaryl, C1-C8 aldehydes, C2-C10 acyl, C2-C10 ester, amino, C1-C6 alkoxy and C1-C10 sulfonyl, etc.

In the present invention, the terms 1-6 refer to 1, 2, 3, 4, 5 or 6. Other similar terms independently have a similar meaning. The term "more" refers to 2-6, such as 2, 3, 4, 5 or 6.

It should be understood that when a certain group is present at a number of different sites in a compound, its definition at each site is independent and may be the same or different. That is, the term "selected from the groups consisting of:" has the same meaning as the term "independently selected from the groups consisting of:".

### Compound

The present invention provides a compound, wherein the compound is a compound shown in Formula I or a stereoisomer, a racemic mixture, or a pharmaceutically acceptable salt,

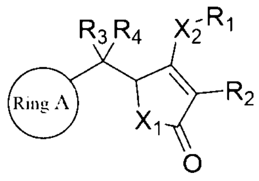

Formula I

wherein, the groups are as defined above.

In another preferred embodiment, any one of A, $X_1$, $X_3$, $R_1$, $R_2$, $R_3$ and $R_4$ is independently a corresponding group in the specific compound described in the present invention.

As used herein, the term "pharmaceutically acceptable salt" refers to a salt of a compound of the invention formed with an acid or base suitable for use as a drug. Pharmaceutically acceptable salts include inorganic and organic salts. A preferably class of salts are salts formed by the compounds of the present invention with acids. Acids suitable for forming salts include, but are not limited to: inorganic acids such as hydrochloric acid, hydrobromic acid, hydrofluoric acid, sulfuric acid, nitric acid, phosphoric acid, and the like; organic acids such as formic acid, acetic acid, trifluoroacetic acid, propionic acid, oxalic acid, malonic acid, succinic acid, fumaric acid, maleic acid, lactic acid, malic acid, tartaric acid, citric acid, picric acid, benzoic acid, methanesulfonic acid, ethylsulfonic acid, p-toluenesulfonic acid, benzene sulfonic acid, naphthalenesulfonic acid, and the like; and amino acids such as proline, phenylalanine, aspartic acid and glutamic acid, etc.

Another preferably class of salts are formed by the compounds of the present invention with bases, such as alkali metal salts (e.g., sodium or potassium salts), alkaline earth metal salts (e.g., magnesium or calcium salts), ammonium salts (e.g., lower alkanolammonium salts, and other pharmaceutically acceptable amine salts), for example, methylamine salts, ethylamine salts, propylamine salts, dimethylamine salts, trimethylamine salts, diethylamine salts, triethylamine salts, tert-butylamine salts, ethylene diamine salts, hydroxyethylamine salts, dihydroxyethylamine salts, trihydroxyethylamine salt and amine salts formed from morpholine, piperazine, and lysine, respectively.

### Preparation Method

The following schemes and examples describe methods for preparing compounds of Formula I. Raw materials and intermediates were purchased from commercial sources, prepared by known steps, or otherwise described. In some cases, the order of the steps in the reaction scheme may be altered to promote the reaction or avoid unwanted by-products.

The preparation method of the compound of Formula I of the present invention are described more specifically below, but these specific methods do not constitute any limitation of the present invention. The compounds of the present invention may also be conveniently prepared by optionally combining various synthetic methods described in this specification or known in the art, and such combinations may be easily carried out by one skilled in the art to the invention.

Typically, in the preparation process, each reaction is carried out under inert gas protection, in an appropriate solvent, at 0 to 150 °C, and the reaction time is usually 2-24 h.

The preferred preparation method is as follows:

Method 1:

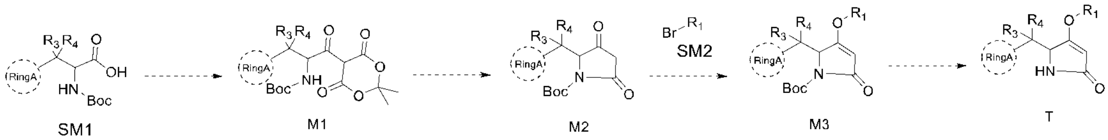


SM1 M1 M2 M3 T

Step 1: In a solvent (dichloromethane), in the present of a condensing agent (DCC, EDCI, etc.) and a base (4-dimethylaminopyridine, diisopropylethylamine), SM1 reacts with Michi acid to form compound M1.

Step 2: In an ultra-dry solvent (ethyl acetate, 1,4-dioxane, etc.), compound M1 reacts under reflux conditions to form compound M2.

Step 3: In a solvent (acetonitrile, toluene, N,N-dimethylformamide, etc.) under basic condition (e.g., diisopropylethylamine, potassium carbonate, DBU, etc.), compound M2 reacts with SM2 to form compound M3.

Step 4: In a solvent (ethyl acetate, dichloromethane, 1,4-dioxane, etc.), under acidic condition (hydrochloric acid, trifluoroacetic acid, etc.), compound M3 reacts to form T (i.e., compound I).

Method 2:

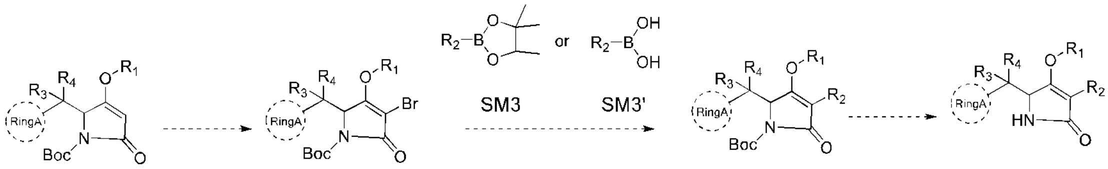


M3 M4 M5 T

Step 1: In a solvent (such as dichloromethane or carbon tetrachloride), compound M3 reacts with N- Bromosuccinimide (NBS) to form compound M4.

Step 2: In an inert solvent (e.g., N,N-dimethylformamide, dioxane, dimethyl sulfoxide, etc.), under basic condition (e.g., potassium carbonate, potassium phosphate, etc.), in the presence of a catalyst and ligand (e.g., $Pd(PPh_3)_4$), compound M4 reacts with SM3 or SM3' to form M5.

Step 3: In a solvent (e.g., ethyl acetate, dichloromethane, 1,4-dioxane, etc.) under acidic condition (e.g., hydrochloric acid, trifluoroacetic acid, etc.), compound M5 reacts to form T (i.e., compound I).

Method 3:

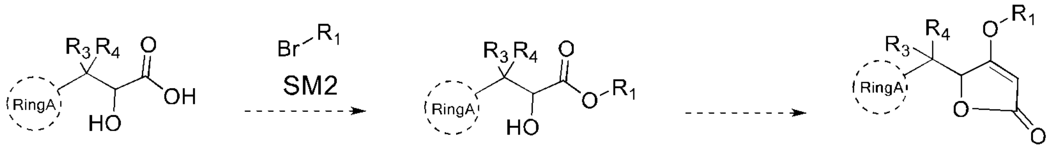


SM4 M6 T

Step 1: In a solvent (e.g., acetonitrile, toluene, N,N-dimethylformamide, etc.), under basic condition (e.g., diisopropylethylamine, potassium carbonate, DBU, etc.), compound SM4 reacts with SM2 to form compound M6.

Step 2: In tetrahydrofuran solvent, under acidic conditions (benzoic acid), compound M6 reacts with (Triphenylphosphoranylidene) ketene to form T (i.e., compound I).

Method 4:

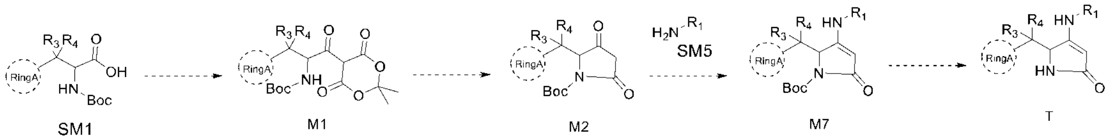

Step 1: In a solvent (dichloromethane), in the present of a condensing agent (DCC, EDCI, etc.) and a base (4-dimethylaminopyridine, diisopropylethylamine), SM1 reacts with Michi acid to form compound M1.

Step 2: In an ultra-dry solvent (ethyl acetate, 1,4-dioxane, etc.), compound M1 reacts under reflux conditions to form compound M2.

Step 3: In a solvent (toluene), under acidic condition (p-toluenesulfonic acid), compound M2 reacts with SM5 to form compound M7.

Step 4: In a solvent (ethyl acetate, dichloromethane, 1,4-dioxane, etc.) under acidic condition (hydrochloric acid, trifluoroacetic acid, etc.), compound M7 reacts to form T (i.e., compound of formula I).

In the above formulas, $R_1$, $R_2$, $R_3$, and $R_4$ are as described above.

Unless otherwise specified, all starting materials can be purchased commercially or synthesized according to previously reported literature.

**Pharmaceutical Composition and Mode of Administration**

The pharmaceutical composition of the present invention comprises a safe and effective amount of a compound of the present invention or a pharmacologically acceptable salt thereof, and a pharmacologically acceptable excipient or carrier. In which, "safe and effective amount" is meant that the amount of the compound is sufficient to significantly improve the condition without causing serious side effects. Generally, the pharmaceutical composition contains 1-2000 mg of the compound of the present invention/dose, more preferably, 10-1000 mg of the compound of the present invention/dose. Preferably, the "dose" is a capsule or tablet.

"Pharmaceutically acceptable carrier" means one or more compatible solid or liquid fillers or gelatinous materials which are suitable for human use and should be of sufficient purity and sufficiently low toxicity. "Compatibility" means that each component in the composition can be admixed with the compounds of the present invention and with each other without significantly reducing the efficacy of the compounds. Some examples of pharmaceutically acceptable carriers include cellulose and the derivatives thereof (such as sodium carboxymethyl cellulose, sodium ethyl cellulose, cellulose acetate, etc.), gelatin, talc, solid lubricants (such as stearic acid, magnesium stearate), calcium sulfate, vegetable oils (such as soybean oil, sesame oil, peanut oil, olive oil, etc.), polyols (such as propylene glycol, glycerol, mannitol, sorbitol, etc.), emulsifiers (such as Tween®), wetting agent (such as sodium dodecyl sulfate), coloring agents, flavoring agents, stabilizers, antioxidants, preservatives, pyrogen-free water, etc..

The pharmaceutical composition is an injection, a capsule, a tablet, a pill, a powder, or a granule.

The administration mode of the compound or pharmaceutical composition of the present invention is not particularly limited, and representative administration modes include, but are not limited to, oral, intratumoral, rectal, parenteral (intravenous, intramuscular or subcutaneous) and topical administration.

Solid dosage forms for oral administration include capsules, tablets, pills, powders and granules. In these solid dosage forms, the active ingredient is mixed with at least one conventional inert excipient (or carrier), such as sodium citrate or dicalcium phosphate, or mixed with any of the following components: (a) fillers or compatibilizer, such as starch, lactose, sucrose, glucose, mannitol and silicic acid; (b) binders, such as hydroxymethyl cellulose, alginate, gelatin, polyvinylpyrrolidone, sucrose and arabic gum; (c) humectants, such as glycerol; (d) disintegrating agents such as agar, calcium carbonate, potato starch or tapioca starch, alginic acid, certain composite silicates, and sodium carbonate; (e) retarding solvents, such as wax, (f) absorption accelerators, such as quaternary ammonium compound; (g) wetting agents, such as cetyl alcohol and glyceryl monostearate; (h) adsorbents, such as kaolin; and (i) lubricants, such as talc, calcium stearate, magnesium stearate, solid polyethylene glycol, sodium dodecyl sulfate or mixture thereof. In capsules, tablets and pills, the dosage forms may also contain buffering agents.

Solid dosage forms such as tablets, dragees, capsules, pills and granules can be prepared with a coating and shell such as enteric coatings and other materials known in the art. They may contain opacifying agents and the release of the active compound or compound in such compositions may be released in a portion of the digestive tract in a delayed manner. Examples of embedding components that can be employed are polymeric materials and waxy materials. If necessary, the active compound may also be in microencapsulated form with one or more of the above-mentioned excipients.

Liquid dosage forms for oral administration include pharmaceutically acceptable emulsions, solutions, suspensions, syrups or tinctures. In addition to the active compound, the liquid dosage form may contain inert diluents conventionally used in the art, such as water or other solvents, solubilizers and emulsifiers, such as ethanol, isopropanol,

ethyl carbonate, ethyl acetate, propylene glycol, 1,3-butanediol, dimethylformamide and oils, especially cottonseed oil, peanut oil, corn germ oil, olive oil, castor oil and sesame oil or mixtures of these substances.

In addition to these inert diluents, the compositions may contain adjuvants such as wetting agents, emulsifying and suspending agents, sweetening agents, flavoring agents and spices.

In addition to the active compound, the suspension may contain suspending agent, such as ethoxylated isooctadecanol, polyoxyethylene sorbitol and dehydrated sorbitan ester, microcrystalline cellulose, aluminum methoxide and agar, or a mixture thereof etc..

The compositions for parenteral injection may comprise physiologically acceptable sterile aqueous or anhydrous solutions, dispersions, suspensions or emulsions, and sterile powders which can be re-dissolved into sterile injectable solutions or dispersions. Suitable aqueous and non-aqueous carriers, diluents, solvents or excipients include water, ethanol, polyols and any suitable mixtures thereof.

Dosage forms for the compounds of the invention for topical administration include ointments, powders, patches, sprays and inhalants. The active ingredient is mixed under sterile conditions with a physiologically acceptable carrier and any preservatives, buffers, or propellants which may be required if necessary.

The compounds of the invention can be administered alone or in combination with other pharmaceutically acceptable compounds (e.g., neurodegenerative disease drugs).

The treatment method of the present invention can be administered alone or in combination with other treatment means or therapeutic drugs.

When the pharmaceutical composition is used, a safe and effective amount of the compound of the present invention is administered to a mammal(such as a human) in need of treatment, wherein the dosage at the time of administration is the pharmaceutically effective dosage, for people having a body weight of 60kg, the daily dose is usually 1-2000mg, preferably 50-1000mg. Of course, specific doses should also consider factors such as the administration route, the health of the patient, etc., which are within the skill of the skilled physician.

Compared with the prior art, the present invention has the following main advantages:

(1) The compounds of the present invention have better pharmacokinetic properties;
(2) The compounds of the present invention have better efficacy.

The present invention will be further illustrated below with reference to the specific examples. It should be understood that these examples are only to illustrate the invention but not to limit the scope of the invention. Experimental methods in which the specific conditions are not specified in the following examples are usually in accordance with conventional conditions such as the conditions described in Sambrook et al., Molecular Cloning: Laboratory Manual (New York: Cold Spring Harbor Laboratory Press, 1989), or in accordance with the conditions recommended by the manufacturer. Unless indicated otherwise, percentage and parts are calculated by weight.

Unless otherwise defined, all professional and scientific terminology used in the text have the same meanings as known to the skilled in the art. In addition, any methods and materials similar or equal with the recorded content can apply to the methods of the invention. The preferred embodiments and the materials described herein are only for demonstration purposes.

**Example** 1

Compounds synthesized of the invention:

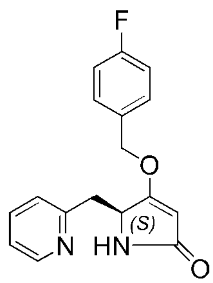

T-01

The experimental process is as follows:
The synthesis route is as follows:

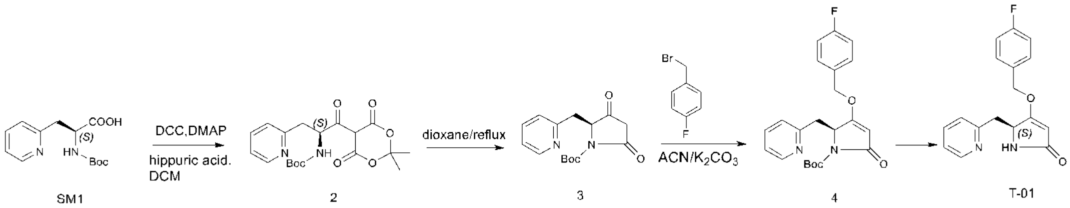


SM1 2 3 4 T-01

1. Synthesis of Compound 2

Compound SM1 (5 g, 1.0 eq), hippuric acid (2.98 g, 1.1 eq), DMAP (3.4 g, 1.5 eq), and solvent DCM (100 ml) were mixed thoroughly in a 100 ml three-neck flask, the mixture was displaced with nitrogen gas three times, and stirred under an ice bath and nitrogen protection for 10 minutes. The internal temperature was kept at 0-10°C, 20 mL of DCM solution containing DCC (4.2 g, 1.1 eq) was added dropwise, and reacted at room temperature for 16 h. TLC detection showed that the raw material reaction was complete. The reaction mixture was filtered, and the filtrate was washed six times with 5% potassium hydrogen sulfate aqueous solution, each time with 50 mL. The organic phase was dried over anhydrous sodium sulfate, and the solvent was evaporated under reduced pressure to obtain a white solid. Then 50 mL petroleum ether was added, stirred thoroughly, and filtered to obtain the target product as a brown solid (3.2 g, yield 43.4%), which was used for the next step without further purification. LC-MS[M-1]: 391.1.

2. Synthesis of Compound 3

Compound 2 (3.2 g, 1.0 eq) was placed in a 100 ml three-neck flask, displaced three times with nitrogen gas, protected with nitrogen gas, and added 45 ml ultra-dry 1,4-dioxane. The mixture was reacted at 100°C for 2 hours. TLC detection showed that the raw material reaction was complete. The reaction mixture was concentrated under reduced pressure to yield 2.6 g of compound 3, no further purification was required for the next step. LC-MS [M-1]: 289.1. Added 50 mL petroleum ether, stirred thoroughly, and filtered to obtain the target product as a brown solid (3.2 g, yield 43.4%), which was used for the next step without further purification. LC-MS[M-1]: 391.1.

3. Synthesis of Compound 4

Compound 3 (1.0 g, 1.0 eq), 4-fluorobenzyl bromide (520 μl, 1.2 eq), potassium carbonate (714 mg, 1.5 eq), and acetonitrile (15 ml) were mixed thoroughly in a 100-ml three-neck flask and refluxed for 2 h. TLC detection showed that the raw material reaction was complete. The solid was removed by vacuum filtration, and the filtrate was purified by column chromatography to obtain 120 mg of compound 4. LC-MS [M+1]: 399.1.

4. Synthesis of compound T-01

Compound 4 (120 mg, 1.0 eq) was added to a 100 ml round-bottom flask and dissolved in DCM (2.5 ml) with stirring, then added trifluoroacetic acid (120 μl, 5.0 eq) dropwise, and reacted at room temperature for 4 h. TLC detection showed that the raw material reaction was complete. The reaction mixture was adjusted to a pH of 7-8 with saturated $Na_2CO_3$, then extracted with dichloromethane. The organic phase was dried over anhydrous sodium sulfate, and the solvent was evaporated under reduced pressure, then purified using a preparative plate to obtain 60 mg of compound T-01. HPLC purity: 99.7%. LC-MS[M+1]: 299.1. 1H NMR (400 MHz, Chloroform-d) δ 8.52 (dt, J = 4.9, 1.5 Hz, 1H), 7.61 (td, J = 7.7, 1.9 Hz, 1H), 7.42 - 7.35 (m, 2H), 7.21 - 7.07 (m, 4H), 6.23 (s, 1H), 5.13 (d, J = 1.7 Hz, 1H), 5.06 - 4.91 (m, 2H), 4.56 (dd, J = 10.5, 3.1 Hz, 1H), 3.38 (dd, J = 14.9, 3.1 Hz, 1H), 2.83 (dd, J = 14.8, 10.4 Hz, 1H).

The following compounds were synthesized according to the method of compound T-01:

| Compound NO. | Compound Structure | characteristic data |
|---|---|---|
| T-02 | 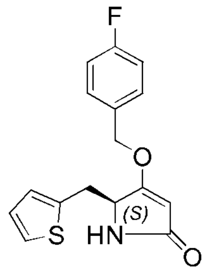 | $^1$H NMR (400 MHz, $CDCl_3$) δ 7.43 - 7.34 (m, 2H), 7.18 (dd, *J* = 5.1, 1.1 Hz, 1H), 7.14 - 7.07 (m, 2H), 6.94 (dd, *J* = 5.1, 3.5 Hz, 1H), 6.85 (d, *J* = 2.9 Hz, 1H), 5.62 (s, 1H), 5.07 (d, *J* = 0.8 Hz, 1H), 4.94 (q, *J* = 11.3 Hz, 2H), 4.29 (dd, *J* = 8.4, 3.3 Hz, 1H), 3.39 (dd, *J* = 14.8, 3.3 Hz, 1H), 2.98 (dd, *J* = 14.8, 8.5 Hz, 1H). |
| T-03 | 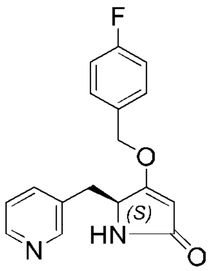 | $^1$H NMR (400 MHz, MeOD) δ 8.39 (dd, J = 4.9, 1.4 Hz, 1H), 8.34 (d, J = 1.6 Hz, 1H), 7.67 - 7.61 (m, 1H), 7.52 - 7.47 (m, 2H), 7.33 (dd, J = 7.5, 4.9 Hz, 1H), 7.20 - 7.13 (m, 2H), 5.03 (t, J = 5.7 Hz, 2H), 4.95 (s, 1H), 4.51 (t, J = 4.8 Hz, 1H), 3.12 (dd, J = 14.1, 4.7 Hz, 1H), 3.03 (dd, J = 14.1, 5.0 Hz, 1H). |
| T-04 | 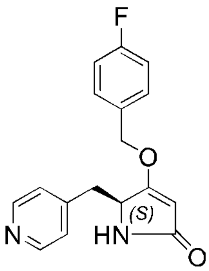 | $^1$H NMR (400 MHz, MeOD) δ 8.38 (dd, J = 4.5, 1.5 Hz, 2H), 7.51 - 7.44 (m, 2H), 7.24 (dd, J = 4.6, 1.5 Hz, 2H), 7.20 - 7.13 (m, 2H), 5.07 (d, J = 3.6 Hz, 1H), 5.06 - 4.98 (m, 2H), 4.53 (t, J = 5.2 Hz, 1H), 3.14 (dd, J = 13.8, 4.8 Hz, 1H), 2.99 (dd, J = 13.8, 5.6 Hz, 1H). |
| T-06 | 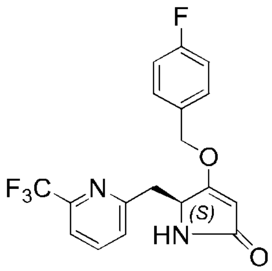 | $^1$H NMR (400 MHz, $CDCl_3$) δ 7.80 (t, J = 7.8 Hz, 1H), 7.57 (d, J = 7.7 Hz, 1H), 7.44 - 7.31 (m, 3H), 7.17 - 7.05 (m, 2H), 6.07 (s, 1H), 5.13 (d, J = 1.1 Hz, 1H), 5.02 - 4.92 (m, 2H), 4.63 (dd, J = 9.7, 2.6 Hz, 1H), 3.45 (dd, J = 15.3, 3.2 Hz, 1H), 2.99 (dd, J = 15.3, 9.7 Hz, 1H). |
| T-07 | 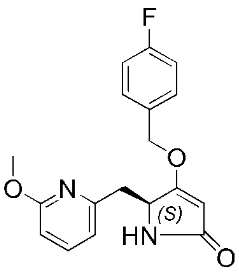 | $^1$H NMR (400 MHz, Chloroform-d) δ 7.50 (dd, J = 8.3, 7.2 Hz, 1H), 7.43 - 7.34 (m, 2H), 7.16 - 7.06 (m, 2H), 6.72 (dd, J = 7.2, 0.8 Hz, 1H), 6.67 - 6.59 (m, 1H), 6.17 (s, 1H), 5.14 (dd, J = 1.7, 0.8 Hz, 1H), 5.06 - 4.92 (m, 2H), 4.57 - 4.49 (m, 1H), 3.92 (s, 3H), 3.30 (dd, J = 14.8, 2.8 Hz, 1H), 2.73 (dd, J = 14.8, 10.6 Hz, 1H). |

(continued)

| Compound NO. | Compound Structure | characteristic data |
|---|---|---|
| T-13 | 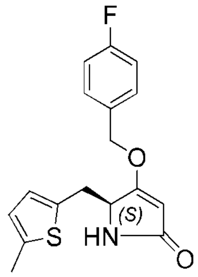 | $^1$H NMR (400 MHz, $CDCl_3$) δ 7.36 (dd, J = 16.2, 10.8 Hz, 2H), 7.11 (t, J = 8.5 Hz, 2H), 6.65 - 6.52 (m, 2H), 5.47 (s, 1H), 5.08 (s, 1H), 5.00 - 4.90 (m, 2H), 4.24 (d, J = 6.2 Hz, 1H), 3.31 (dd, J = 14.8, 2.6 Hz, 1H), 2.86 (dd, J = 14.8, 8.8 Hz, 1H), 2.43 (s, 3H). |
| T-14 | 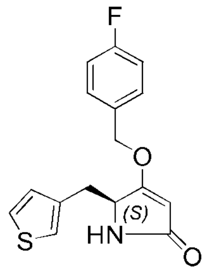 | $^1$H NMR (400 MHz, $CDCl_3$) δ 7.43 - 7.34 (m, 2H), 7.18 (dd, J = 5.1, 1.1 Hz, 1H), 7.14 - 7.07 (m, 2H), 6.94 (dd, J = 5.1, 3.5 Hz, 1H), 6.85 (d, J = 2.9 Hz, 1H), 5.62 (s, 1H), 5.07 (d, J = 0.8 Hz, 1H), 4.94 (q, J = 11.3 Hz, 2H), 4.29 (dd, J = 8.4, 3.3 Hz, 1H), 3.39 (dd, J = 14.8, 3.3 Hz, 1H), 2.98 (dd, J = 14.8, 8.5 Hz, 1H). |
| T-33 | 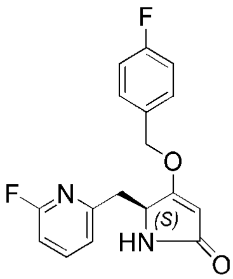 | $^1$H NMR (400 MHz, $CDCl_3$) δ 7.71 (dd, J = 15.7, 8.0 Hz, 1H), 7.37 (dd, J = 8.5, 5.3 Hz, 2H), 7.10 (t, J = 8.6 Hz, 2H), 7.03 (dt, J = 11.5, 5.7 Hz, 1H), 6.81 (dd, J = 8.2, 2.6 Hz, 1H), 6.00 (s, 1H), 5.13 (s, 1H), 4.97 (q, J = 11.4 Hz, 2H), 4.54 (dd, J = 9.9, 2.8 Hz, 1H), 3.33 (dd, J = 14.8, 3.1 Hz, 1H), 2.82 (dd, J = 14.8, 10.0 Hz, 1H). |
| T-34 | 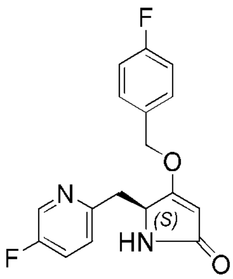 | $^1$H NMR (400 MHz, $CDCl_3$) δ 8.38 (d, J = 2.9 Hz, 1H), 7.45 - 7.30 (m, 3H), 7.21 - 7.02 (m, 3H), 6.21 (s, 1H), 5.13 (d, J = 1.2 Hz, 1H), 4.98 (q, J = 11.4 Hz, 2H), 4.53 (dd, J = 10.1, 3.0 Hz, 1H), 3.36 (dd, J = 14.9, 3.0 Hz, 1H), 2.84 (dd, J = 14.9, 10.2 Hz, 1H). |
| T-35 | 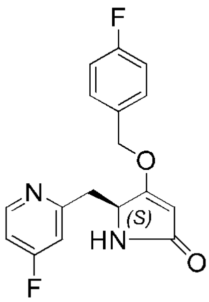 | $^1$H NMR (400 MHz, $CDCl_3$) δ 8.47 (dd, J = 8.5, 5.7 Hz, 1H), 7.36 (dd, J = 8.2, 5.4 Hz, 2H), 7.09 (t, J = 8.6 Hz, 2H), 6.99 - 6.81 (m, 2H), 6.36 (s, 1H), 5.12 (s, 1H), 4.96 (q, J = 11.4 Hz, 2H), 4.54 (dd, J = 9.9, 2.4 Hz, 1H), 3.35 (dd, J = 15.0, 2.9 Hz, 1H), 2.84 (dd, J = 14.9, 10.1 Hz, 1H). |

(continued)

| Compound NO. | Compound Structure | characteristic data |
|---|---|---|
| T-36 | 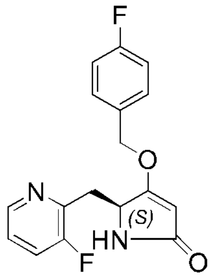 | $^{1}H$ NMR (400 MHz, DMSO) δ 8.37 - 8.30 (m, 1H), 7.64 - 7.54 (m, 2H), 7.45 - 7.38 (m, 2H), 7.32 (dt, J = 8.6, 4.4 Hz, 1H), 7.25 - 7.18 (m, 2H), 5.12 (d, J = 0.9 Hz, 1H), 4.97 (q, J = 11.8 Hz, 2H), 4.49 (t, J = 6.4 Hz, 1H), 3.14 (ddd, J = 14.1, 5.5, 2.0 Hz, 1H), 2.95 (ddd, J = 14.2, 7.3, 2.1 Hz, 1H). |
| T-37 | 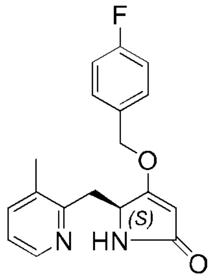 | $^{1}H$ NMR (400 MHz, $CDCl_3$) δ 8.35 (d, J = 4.3 Hz, 1H), 7.43 (d, J = 7.2 Hz, 1H), 7.38 (dd, J = 8.5, 5.4 Hz, 2H), 7.15 - 7.06 (m, 3H), 6.46 (s, 1H), 5.16 (s, 1H), 5.05 - 4.95 (m, 2H), 4.73 (d, J = 9.0 Hz, 1H), 3.37 (dd, J = 16.4, 2.5 Hz, 1H), 2.73 (dd, J = 16.3, 11.0 Hz, 1H), 2.26 (s, 3H). |
| T-41 | 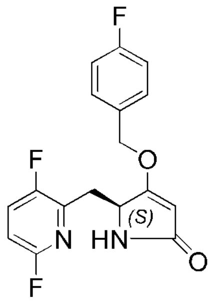 | $^{1}H$ NMR (400 MHz, $CDCl_3$) δ 7.50 - 7.33 (m, 3H), 7.10 (t, J = 8.6 Hz, 2H), 6.81 (dt, J = 8.6, 3.2 Hz, 1H), 6.04 (s, 1H), 5.13 (s, 1H), 5.05 - 4.88 (m, 2H), 4.56 (dd, J = 9.2, 3.3 Hz, 1H), 3.53 - 3.27 (m, 1H), 2.88 (ddd, J = 15.7, 9.3, 1.8 Hz, 1H). |
| T-42 | 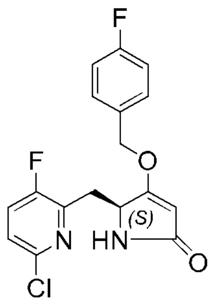 | $^{1}H$ NMR (400 MHz, $CDCl_3$) δ 7.43 - 7.29 (m, 3H), 7.21 (dd, J = 8.6, 3.3 Hz, 1H), 7.11 (t, J = 8.6 Hz, 2H), 6.05 (s, 1H), 5.13 (s, 1H), 5.03 - 4.92 (m, 2H), 4.59 (dd, J = 9.1, 3.3 Hz, 1H), 3.50 - 3.38 (m, 1H), 2.90 (ddd, J = 15.7, 9.2, 1.8 Hz, 1H). |
| T-43 | 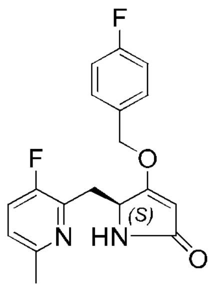 | $^{1}H$ NMR (400 MHz, $CDCl_3$) δ 7.38 (dd, J = 8.5, 5.3 Hz, 2H), 7.23 (t, J = 8.8 Hz, 1H), 7.11 (dd, J = 12.0, 5.3 Hz, 2H), 7.02 (dd, J = 8.4, 3.7 Hz, 1H), 6.33 (s, 1H), 5.13 (d, J = 1.2 Hz, 1H), 5.04 - 4.91 (m, 2H), 4.57 (dd, J = 10.1, 2.9 Hz, 1H), 3.46 (dt, J = 15.8, 2.6 Hz, 1H), 2.81 (ddd, J = 15.8, 10.2, 1.6 Hz, 1H), 2.49 (s, 3H). |

(continued)

| Compound NO. | Compound Structure | characteristic data |
|---|---|---|
| T-46 | 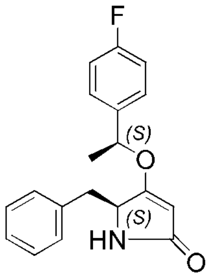 | $^{1}H$ NMR (400 MHz, Chloroform-d) δ 7.36 - 7.28 (m, 5H), 7.25 - 7.21 (m, 2H), 7.11 - 7.02 (m, 2H), 5.30 - 5.22 (m, 1H), 5.11 (q, J = 6.4 Hz, 1H), 4.82 (d, J = 1.7 Hz, 1H), 4.19 (dt, J = 9.5, 2.3 Hz, 1H), 3.27 (dd, J = 13.6, 3.6 Hz, 1H), 2.67 (dd, J = 13.6, 9.5 Hz, 1H), 1.65 (d, J = 6.5 Hz, 3H). |
| T-47 | 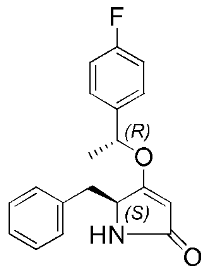 | $^{1}H$ NMR (400 MHz, Chloroform-d) δ 7.34 - 7.27 (m, 5H), 7.21 - 7.16 (m, 2H), 7.11 - 7.04 (m, 2H), 5.24 (s, 1H), 5.16 (q, J = 6.4 Hz, 1H), 4.85 (d, J = 1.7 Hz, 1H), 4.29 - 4.19 (m, 1H), 3.27 (dd, J = 13.5, 3.5 Hz, 1H), 2.46 (dd, J = 13.5, 10.3 Hz, 1H), 1.66 (d, J = 6.5 Hz, 3H). |
| T-51 | 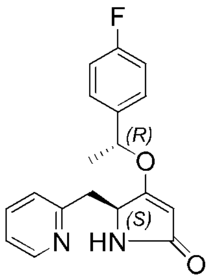 | $^{1}H$ NMR (400 MHz, $CDCl_3$) δ 8.43 (d, J = 4.7 Hz, 1H), 7.75 (dd, J = 7.6, 6.3 Hz, 1H), 7.36 (dt, J = 5.2, 4.4 Hz, 2H), 7.28 (dd, J = 16.6, 7.2 Hz, 2H), 6.89 (t, J = 8.3 Hz, 2H), 5.65 (s, 1H), 4.20 (dd, J = 17.0, 12.1 Hz, 1H), 3.95 - 3.84 (m, 1H), 3.26 - 3.17 (m, 2H), 1.57 (d, J = 6.8 Hz, 3H). |
| T-89 | 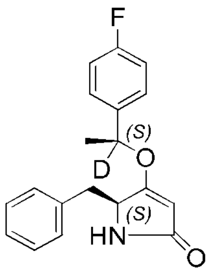 | LC-MS[M+1]: 313.1 |
| T-90 | 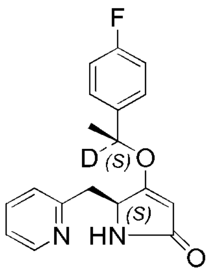 | LC-MS[M+1]: 314.1 |

(continued)

| Compound NO. | Compound Structure | characteristic data |
|---|---|---|
| T-91 | 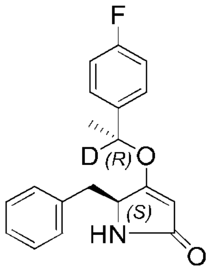 | LC-MS[M+1]: 313.1 |
| T-92 | 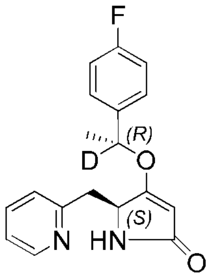 | LC-MS[M+1]: 314.1 |
| T-105 | 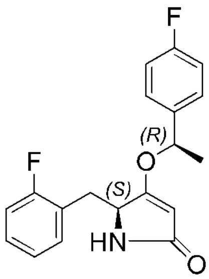 | LC-MS[M+1]: 330.1 |
| T-106 | 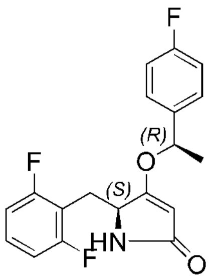 | LC-MS[M+1]: 348.1 |
| T-113 | 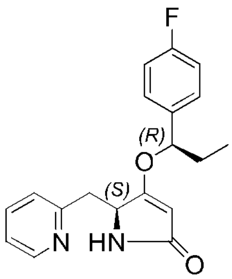 | LC-MS[M+1]: 327.1 |

(continued)

| Compound NO. | Compound Structure | characteristic data |
| --- | --- | --- |
| T-114 | 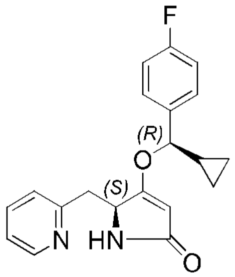 | LC-MS[M+1]: 339.1 |
| T-115 | 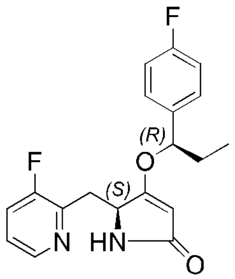 | LC-MS[M+1]: 345.1 |
| T-116 | 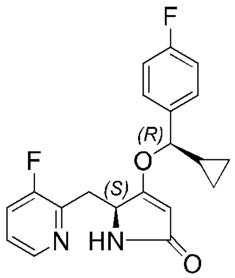 | LC-MS[M+1]: 357.1 |
| T-117 | 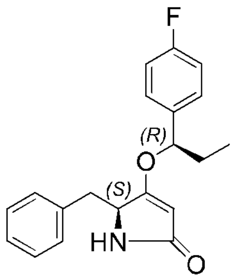 | LC-MS[M+1]: 326.1 |
| T-118 | 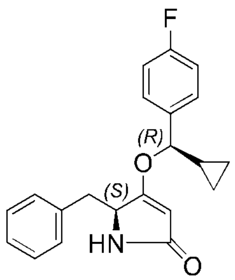 | LC-MS[M+1]: 338.1 |

(continued)

| Compound NO. | Compound Structure | characteristic data |
|---|---|---|
| T-119 | 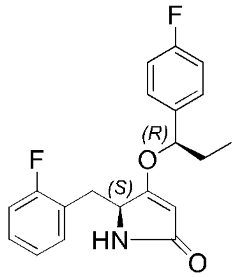 | LC-MS[M+1]: 344.1 |
| T-120 | 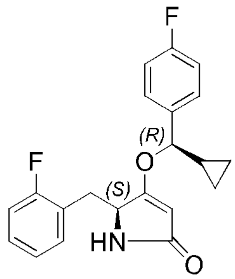 | LC-MS[M+1]: 356.1 |
| T-137 | 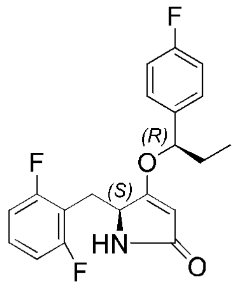 | LC-MS[M+1]: 362.1 |
| T-138 | 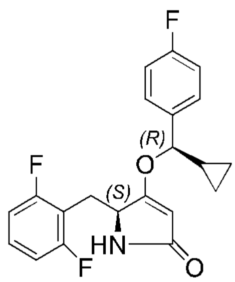 | LC-MS[M+1]: 374.1 |

**Example 2**

Compounds synthesized of the invention:

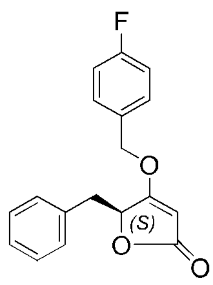

T-29

The experimental process is as follows:
The synthesis route is as follows:

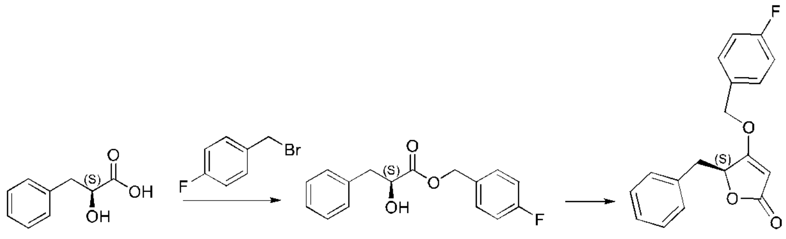

SM1 2 T-29

1. Synthesis of Compound 2

Compound SM1 (1 g, 1.0 eq), 4-fluorobenzene bromide (1.14 g, 1.0 eq), DBU (1.2 g, 1.3 eq), and acetonitrile (15 ml) were added to a 100 ml three-neck flask and mixed thoroughly. The mixture was displaced with nitrogen gas three times, and reacted at room temperature under nitrogen protection. TLC detection showed that the raw material reaction was complete. The solvent was removed under reduced pressure, EA and water were added for two-phase extraction, then the EA layer was washed with saturated NaCl solution, the EA layer was dried over anhydrous sodium sulfate, and EA was removed by rotary evaporation to obtain 1.2 g product. $^1$H NMR (400 MHz, DMSO) δ 7.34 (dd, J = 8.6, 5.7 Hz, 2H), 7.28 - 7.13 (m, 7H), 5.07 (s, 2H), 4.29 (dt, J = 7.7, 5.7 Hz, 1H), 2.96 (dd, J = 13.7, 5.3 Hz, 1H), 2.85 (dd, J = 13.7, 7.8 Hz, 1H).

2. Synthesis of compound T-29

Compound 2 (300 mg, 1.0 eq), (Triphenylphosphoranylidene)ketene (378 mg, 1.1 eq), benzoic acid (12.5 mg, 0.1 eq) and THF (9 ml) were added to a 100 ml three-neck flask and mixed thoroughly, the mixture was displaced with nitrogen gas three times, and protected with nitrogen gas. The reaction was carried out at room temperature, and TLC detection showed that the raw material reaction was complete. EA and water were added for two-phase extraction, the EA layer was washed with saturated NaCl solution, the EA layer was dried over anhydrous sodium sulfate, and EA was removed by rotary evaporation to obtain 700 mg of product. After purification, 87 mg of product was obtained with HPLC purity 97.7%.
$^1$H NMR (400 MHz, DMSO) δ 7.69 - 7.55 (m, 2H), 7.45 - 7.26 (m, 5H), 7.21 (dd, *J* = 12.6, 6.2 Hz, 2H), 5.44 (s, 1H), 5.31 (dd, *J* = 5.7, 4.4 Hz, 1H), 5.17 (dd, *J* = 33.8, 11.7 Hz, 2H), 3.24 (dd, *J* = 14.5, 4.1 Hz, 1H), 2.93 (dd, *J* = 14.5, 6.4 Hz, 1H).

**Example 3**

Compounds synthesized of the invention:

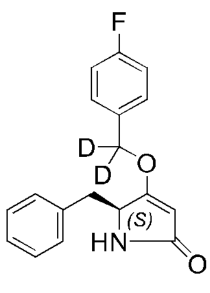

T-45

The experimental process is as follows:

(1) Synthesis of intermediate SM2

The synthesis route is as follows:

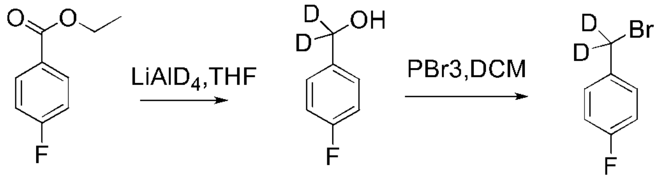


1 2 SM2

1. Synthesis of Compound 2

Compound 1 (1 g, 1.0 eq) was added to a 100 ml three-neck flask, displaced three times with nitrogen gas, protected with nitrogen gas and dissolved in ultra-dry THF (30 ml), stirred in an ice bath, maintained at a temperature of 0-10°C, then lithium aluminum deuteride (476 mg, 2.0 eq) was added in batches, and reacted at room temperature overnight. TLC detection showed that the raw material reaction was complete, quenched the reaction with saturated ammonium chloride solution under ice bath, filtered with diatomaceous earth, dried over anhydrous sodium sulfate, and spun-dried to obtain 780 mg of colorless liquid. $^1$H NMR (400 MHz, Chloroform-d) δ 7.38 - 7.30 (m, 2H), 7.09 - 7.00 (m, 2H).

2. Synthesis of compound SM2

Compound 2 (780 mg, 1.0 eq) and dichloromethane (25 ml) were added to a 100 ml three-neck flask , the mixture was displaced three times with nitrogen gas and protected with nitrogen gas, then stirred under an ice bath, added phosphorus tribromide (638 ul, 1.1 eq) dropwise, and reacted at room temperature for 3 hours. TLC detection showed that the raw material reaction was complete. Quenched with water, extracted with dichloromethane, the organic phase was washed with saturated sodium chloride solution, dried over anhydrous sodium sulfate, and purified by column chromatography to obtain 800 mg of compound SM2. $^1$H NMR (400 MHz, Chloroform-d) δ 7.43 - 7.32 (m, 2H), 7.09 - 6.97 (m, 2H).

(2) Synthesis of Compound T-45

The synthesis route is as follows:

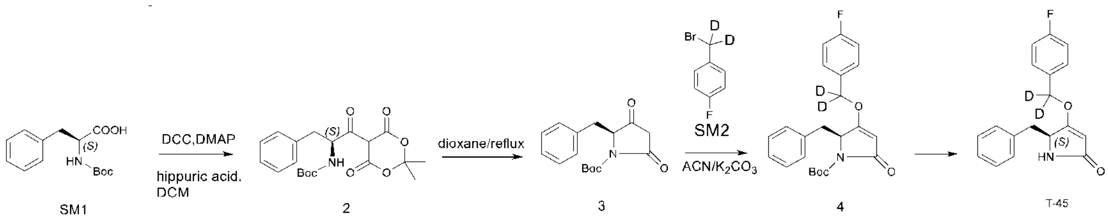


SM1 2 3 4 T-45

Compound T-45 was obtained by referring to Example 1. $^1$H NMR (400 MHz, Chloroform-d) δ 7.43 - 7.35 (m, 2H), 7.34 - 7.26 (m, 3H), 7.22 - 7.16 (m, 2H), 7.15 - 7.07 (m, 2H), 5.45 - 5.32 (m, 1H), 5.07 (d, J = 1.6 Hz, 1H), 4.26 (dd, J = 9.6, 3.6 Hz, 1H), 3.23 (dd, J = 13.6, 3.6 Hz, 1H), 2.63 (dd, J = 13.6, 9.6 Hz, 1H).

The following compounds were synthesized by referring to the method of compound T-45:

| Compound NO. | Compound Structure | characteristic data |
|---|---|---|
| T-10 | 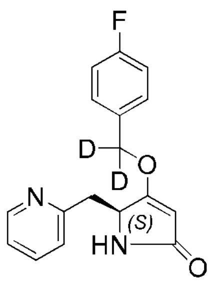 | $^1$H NMR (400 MHz, Chloroform-d) δ 8.52 (ddd, J = 4.9, 2.0, 1.0 Hz, 1H), 7.61 (td, J = 7.7, 1.9 Hz, 1H), 7.43 - 7.35 (m, 2H), 7.21 - 7.06 (m, 4H), 6.24 (s, 1H), 5.13 (dd, J = 1.8, 0.8 Hz, 1H), 4.56 (ddt, J = 10.5, 3.0, 1.1 Hz, 1H), 3.38 (dd, J = 14.9, 3.0 Hz, 1H), 2.83 (dd, J = 14.9, 10.5 Hz, 1H). |

**Example 4**

Compounds synthesized of the invention:

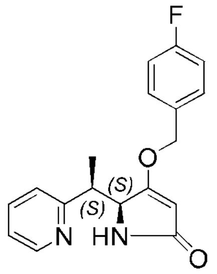
、
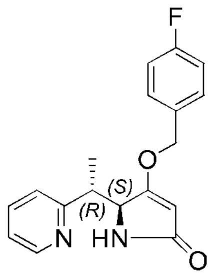

T-73 T-74

The experimental process is as follows:

(1) Synthesis of intermediate SM1

The synthesis route is as follows:

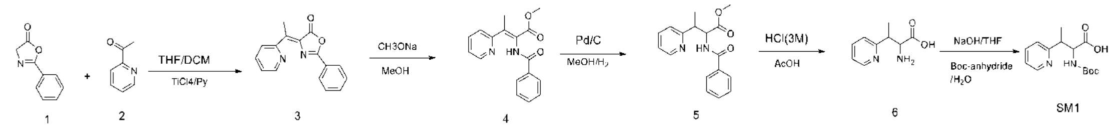


1. Synthesis of compound 3

50 ml of THF was added to a 250 ml three-neck flask, displaced three times with nitrogen gas, protected with nitrogen gas, and cooled to -10°C in an ice-salt bath, then added $TiCl_4$ (4.64 ml, 1.5 eq) and DCM (1 ml) mixture dropwise and stirred for 20 min in an ice-salt bath after dropping, added compound 1 (3.3 ml, 1.0 eq) and stirred for 10 min under ice-salt bath, added compound 2 (5 g, 1.1 eq) and stirred for 30 min under ice-salt bath, and added pyridine (4.5 ml, 2.0 eq), then raised to room temperature and reacted overnight. TLC detection showed that the raw material reaction was complete. Quenched with saturated $NH_4Cl_4$ solution under ice bath, extracted with EA five times, the EA layer washed with saturated sodium chloride solution, dried over anhydrous sodium sulfate, and purified by column chromatography to obtain 5.5 g of compound 3, LC-MS [M+1]: 265.1.

2. Synthesis of compound 4

Compound 3 (5.5 g, 1.0 eq) and MeOH (55 ml) were added to a 250 ml round-bottom flask, displaced three times with nitrogen gas and protected with nitrogen gas, then added sodium methoxide (227 mg, 0.2 eq) and reacted overnight at 70°C. TLC detection showed that the raw material reaction was complete. Stopped heating, cooled to room temperature, the solvent was spun-dried, and purified by column chromatography to obtain 4.6 g of compound 4, LC-MS [M+1]: 297.1.

3. Synthesis of compound 5

Compound 4 (4.6 g, 1.0 eq), MeOH (46 ml), and Pd/C (460 mg, 0.1 eq) were added to a 100 ml reactor, displaced three times with hydrogen gas and protected with hydrogen gas, then reacted at 70°C for 5.5 h. Stopped heating, cooled to room temperature, and TLC detection showed that the raw material reaction was complete. After filtration using diatomaceous earth, the filtrate was spun-dried to obtain 5.19 g of compound 5. LC-MS [M+1]: 299.1.

4. Synthesis of compound 6

Compound 5 (4.19 g, 1.0 eq), HCl (3 M) (104 ml, 25.0 eq), and glacial acetic acid (42 ml, 50.0 eq) were added to a 250 ml round-bottom flask, displaced three times with nitrogen gas, and protected with nitrogen gas, then reacted overnight at 125°C. TLC detection showed that the raw material reaction was complete. Stopped heating, cooled to room temperature, then water was added, filtered, and the filtrate was spun-dried to obtained 4.69 g of compound 6, LC-MS

[M+1]: 181.1.

5. Synthesis of compound SM1

To a 250 ml round-bottom flask was added compound 6 (4.69 g, 1.0 eq), THF (47 ml), and $H_2O$ (47 ml), displaced three times with nitrogen gas, protected with nitrogen gas, and stirred in an ice bath for 10 min, then added NaOH (4.2 g, 4.0 eq) and stirred in an ice bath for 5 min, and then added Boc anhydride (6.68 ml, 1.1 eq) and reacted overnight after raising to room temperature. TLC detection showed that the raw material reaction was complete. After removing the solvent THF by steam distillation, an appropriate amount of DCM was added, and the pH was adjusted to 4-5 with 1N HCl. After stirring thoroughly for 10 min, the mixture was separated into layers. The DCM layer was washed with saturated sodium chloride solution, dried over anhydrous sodium sulfate and purified by column chromatography to obtain 2 g of compound SM1, LC-MS [M+1]: 281.1.

(2) Synthesis of Compound T-73, T-74

The synthesis route is as follows:

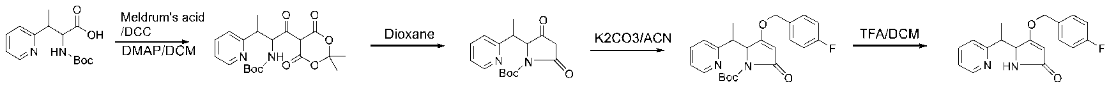


SM1 2 3 4 5

Compound 5 was obtained by referring to Example 1, and after preparation and separation, compounds T-73 and T-74 were obtained. LC-MS[M+1]: 313.1.

The following compounds were synthesized by referring to the method of compound T-73 and T-74:

| Compound NO. | Compound Structure | characteristic data |
|---|---|---|
| T-75 | 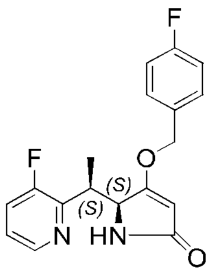 | LC-MS[M+1]: 331.1 |
| T-76 | 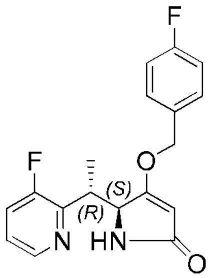 | LC-MS[M+1]: 331.1 |
| T-77 | 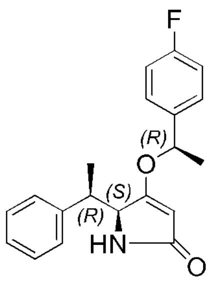 | LC-MS[M+1]: 326.1 |

(continued)

| Compound NO. | Compound Structure | characteristic data |
|---|---|---|
| T-78 | 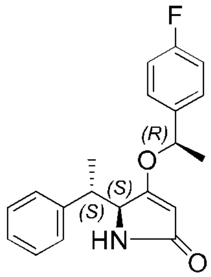 | LC-MS[M+1]: 326.1 |
| T-79 | 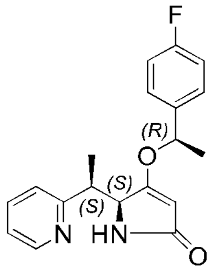 | LC-MS[M+1]: 327.1 |
| T-80 | 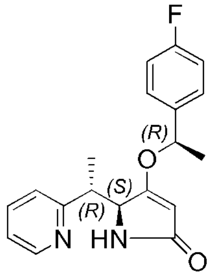 | LC-MS[M+1]: 327.1 |
| T-95 | 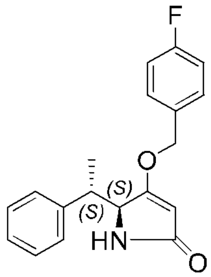 | $^1$H NMR (400 MHz, $CDCl_3$) δ 7.34 (dd, J = 8.4, 5.4 Hz, 2H), 7.28 (d, J = 7.3 Hz, 1H), 7.23 (d, J = 6.9 Hz, 2H), 7.16 (d, J = 6.6 Hz, 2H), 7.11 (t, J = 8.6 Hz, 2H), 5.50 (s, 1H), 4.91 (s, 1H), 4.80 (dd, J = 41.4, 11.3 Hz, 2H), 4.24 (d, J = 5.7 Hz, 1H), 3.17 - 3.08 (m, 1H), 1.43 (d, J = 7.2 Hz, 3H). |
| T-96 | 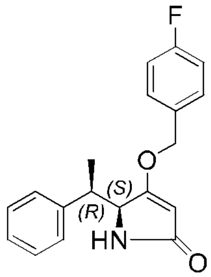 | $^1$H NMR (400 MHz, $CDCl_3$) δ 7.41 - 7.35 (m, 2H), 7.33 (d, J = 7.3 Hz, 2H), 7.27 (s, 1H), 7.23 (s, 2H), 7.11 (t, J = 8.4 Hz, 2H), 5.15 (s, 1H), 5.07 (s, 1H), 4.97 (q, J = 11.3 Hz, 2H), 4.26 (s, 1H), 3.31 (d, J = 3.7 Hz, 1H), 1.14 (d, J = 7.0 Hz, 3H). |

(continued)

| Compound NO. | Compound Structure | characteristic data |
|---|---|---|
| T-97 | 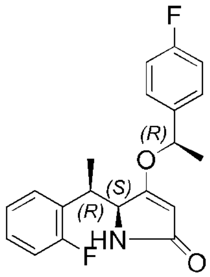 | LC-MS[M+1]: 344.1 |
| T-98 | 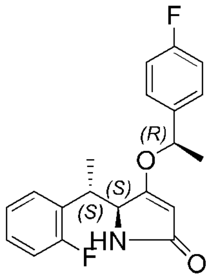 | LC-MS[M+1]: 344.1 |
| T-99 | 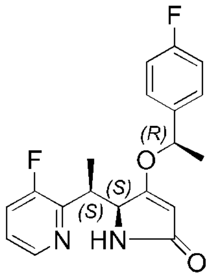 | LC-MS[M+1]: 345.1 |
| T-100 | 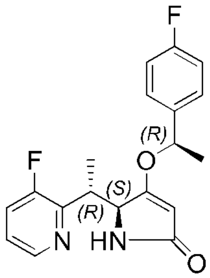 | LC-MS[M+1]: 345.1 |
| T-101 | 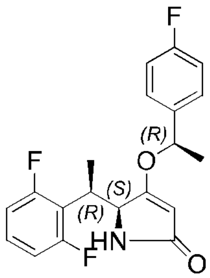 | LC-MS[M+1]: 362.1 |

(continued)

| Compound NO. | Compound Structure | characteristic data |
|---|---|---|
| T-102 | 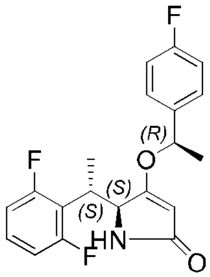 | LC-MS[M+1]: 362.1 |
| T-103 | 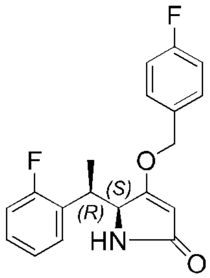 | LC-MS[M+1]: 330.1 |
| T-104 | 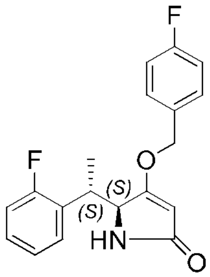 | LC-MS[M+1]: 330.1 |
| T-109 | 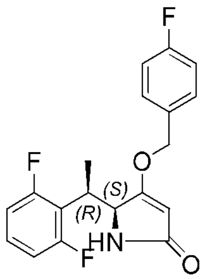 | LC-MS[M+1]: 348.1 |
| T-110 | 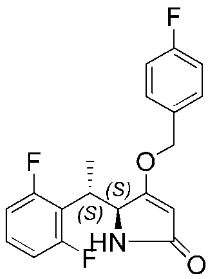 | LC-MS[M+1]: 348.1 |

(continued)

| Compound NO. | Compound Structure | characteristic data |
|---|---|---|
| T-121 | 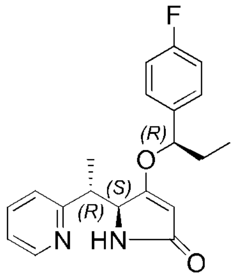 | LC-MS[M+1]: 341.2 |
| T-122 | 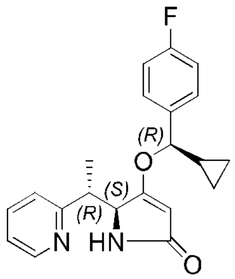 | LC-MS[M+1]: 353.2 |
| T-123 | 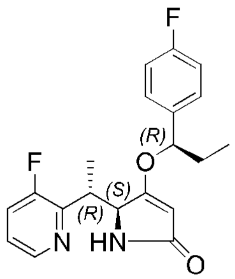 | LC-MS[M+1]: 359.1 |
| T-124 | 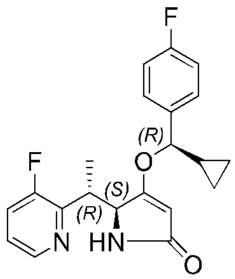 | LC-MS[M+1]: 371.1 |
| T-125 | 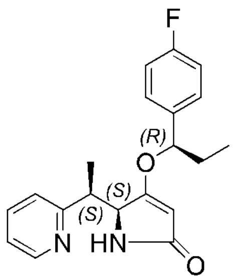 | LC-MS[M+1]: 341.2 |

(continued)

| Compound NO. | Compound Structure | characteristic data |
|---|---|---|
| T-126 | 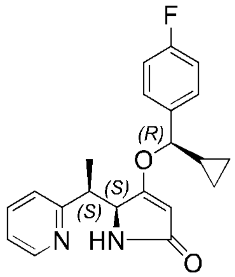 | LC-MS[M+1]: 353.2 |
| T-127 | 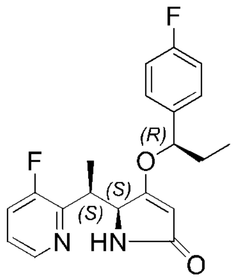 | LC-MS[M+1]: 359.1 |
| T-128 | 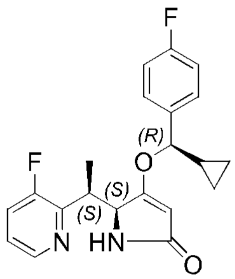 | LC-MS[M+1]: 371.1 |
| T-129 | 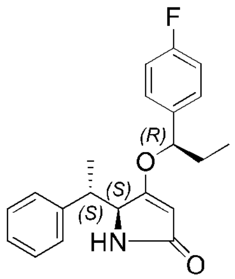 | LC-MS[M+1]: 340.2 |
| T-130 | 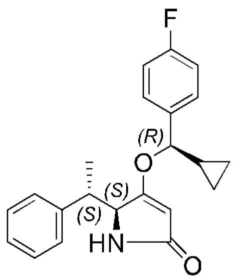 | LC-MS[M+1]: 352.2 |

(continued)

| Compound NO. | Compound Structure | characteristic data |
|---|---|---|
| T-131 | 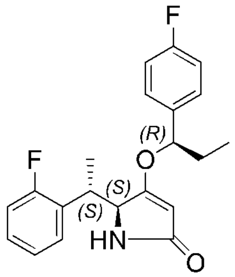 | LC-MS[M+1]: 358.2 |
| T-132 | 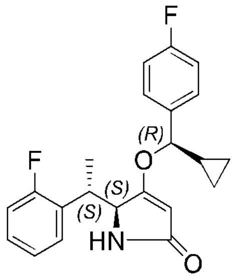 | LC-MS[M+1]: 370.2 |
| T-133 | 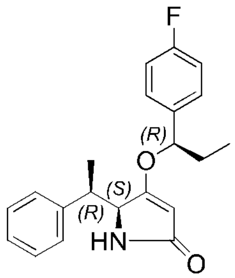 | LC-MS[M+1]: 340.2 |
| T-134 | 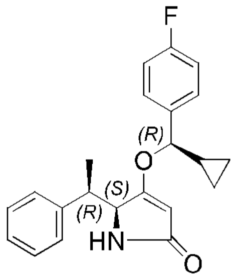 | LC-MS[M+1]: 352.2 |
| T-135 | 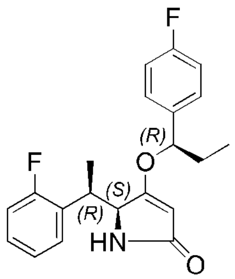 | LC-MS[M+1]: 358.2 |

(continued)

| Compound NO. | Compound Structure | characteristic data |
|---|---|---|
| T-136 | 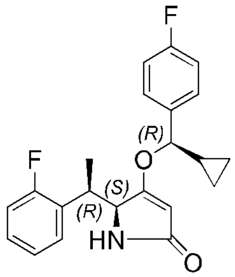 | LC-MS[M+1]: 370.2 |
| T-139 | 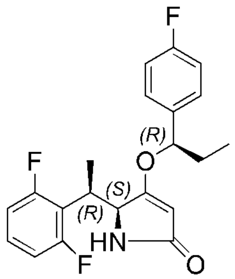 | LC-MS[M+1]: 376.1 |
| T-140 | 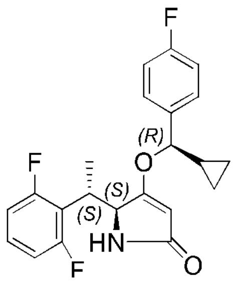 | LC-MS[M+1]: 388.1 |

**Example 5**

Compounds synthesized of the invention:

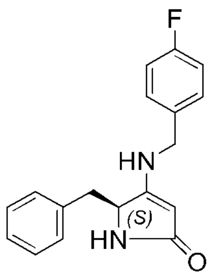

T-81

The experimental process is as follows:
The synthesis route is as follows:

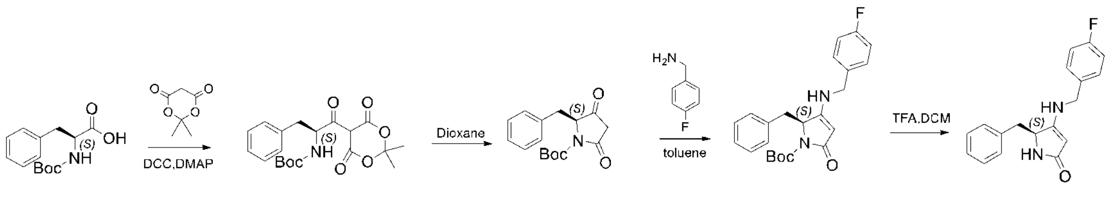


1 2 3 4 T-81

1. Synthesis of compound 2

To a 250 ml three-neck flask was added Compound SM1 (10 g, 1.0 eq), Meldrum's acid (5.97 g, 1.1 eq), DMAP (6.9 g, 1.5 eq) and solvent DCM (100 ml) and mixed evenly. After displacing three times with nitrogen gas, the mixture was protected with nitrogen gas and stirred for 10 min under an ice bath. The internal temperature was controlled at 0-10°C, and 50 ml of DCM solution of DCC (8.55 g, 1.1 eq) was added dropwise then reacted at room temperature for 16 h. TLC detection showed that the raw material reaction was complete. The reaction solution was filtered, and the filtrate was washed with 5% potassium hydrogen sulfate aqueous solution six times, 50 ml each time. The organic phase was dried over anhydrous sodium sulfate. The solvent was evaporated under reduced pressure to obtain a white solid, 50 mL of petroleum ether was added and stirred thoroughly, and filtered to obtain 14.3 g of the target product as a light yellow solid, which was used to next step without further purification. LC-MS[M-1]: 390.1.

2. Synthesis of compound 3

To a 100 ml three-neck flask was added Compound 2 (14.3 g, 1.0 eq), displaced three times with nitrogen gas, protected with nitrogen gas, and ultra-dry 1,4-dioxane (172 ml) was added then reacted at 100°C for 2 hours. TLC detection showed that the raw material reaction was complete. The reaction mixture was concentrated under reduced pressure to obtain 11.2 g of compound 3, which was used to next step without further purification. LC-MS [M-1]: 288.1.

3. Synthesis of compound 4

To a 100 ml three-neck flask was added compound 3 (2.0 g, 1.0 eq), toluene (20 ml), p-fluorobenzylamine (790 ul, 1.0 eq), and p-toluenesulfonic acid (catalytic amount) and mixed evenly, displaced three times with nitrogen gas, and reacted at 100°C for 5 hours under nitrogen protection. TLC detection showed that the raw material reaction was complete. Extracted with water and ethyl acetate, the organic phase was washed with saturated sodium chloride, dried over anhydrous sodium sulfate, and purified by column chromatography to obtain 965 mg of compound 4. LC-MS[M+1]: 397.1. 1H NMR (400 MHz, Chloroform-d) δ 7.24 (td, J = 6.7, 6.3, 3.3 Hz, 3H), 7.19 - 7.15 (m, 2H), 7.10 (dd, J = 8.5, 5.4 Hz, 2H), 7.04 - 6.97 (m, 2H), 4.67 (dd, J = 8.7, 3.3 Hz, 1H), 4.63 (s, 1H), 4.45 (s, 1H), 4.06 (dd, J = 5.3, 1.7 Hz, 2H), 3.52 (dd, J = 13.7, 3.3 Hz, 1H), 2.93 (dd, J = 13.7, 8.7 Hz, 1H), 1.59 (s, 9H).

4. Synthesis of compound T-81

To a 100 ml three-neck flask added compound 4 (100 mg, 1.0 eq) and DCM (2.0 ml)and stirred to dissolve, then added trifluoroacetic acid (193 ul, 10.0 eq) dropwise under an ice bath, and reacted at room temperature for 3h. TLC detection showed that the raw material reaction was complete. Extracted with saturated sodium bicarbonate solution and ethyl acetate. The organic phase was washed with saturated sodium chloride solution, dried over anhydrous sodium sulfate, and purified by preparative separation to obtain compound T-81 (30 mg). HPLC purity: 97.1%.

LC-MS[M+1]:297.1. 1H NMR (400 MHz, Chloroform-d) δ 7.30 (dd, J = 7.8, 6.1 Hz, 2H), 7.25 (s, 1H), 7.23 - 7.17 (m, 4H), 7.02 (t, J = 8.6 Hz, 2H), 5.28 (s, 1H), 4.67 (d, J = 1.5 Hz, 1H), 4.61 (t, J = 5.4 Hz, 1H), 4.23 (dd, J = 8.6, 5.6 Hz, 1H), 4.15 (d, J = 5.3 Hz, 2H), 3.05 - 2.77 (m, 2H).

The following compounds were synthesized by referring to the method of compound T-81:

| Compound NO. | Compound Structure | characteristic data |
|---|---|---|
| T-83 | 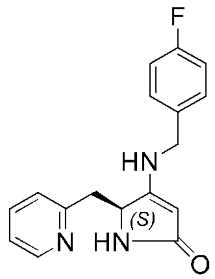 | LC-MS[M+1]: 298.1 |
| T-85 | 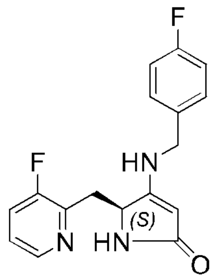 | LC-MS[M+1]: 316.1 |
| T-87 | 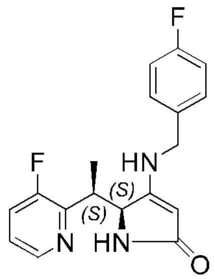 | LC-MS[M+1]: 330.1 |
| T-93 | 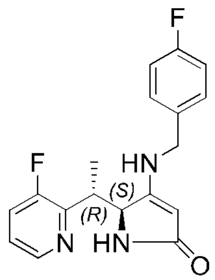 | LC-MS[M+1]: 330.1 |
| T-107 | 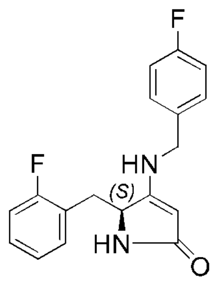 | LC-MS[M+1]: 315.1 |

(continued)

| Compound NO. | Compound Structure | characteristic data |
|---|---|---|
| T-111 | 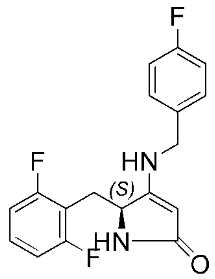 | LC-MS[M+1]: 333.1 |

**Example 6**

Compounds synthesized of the invention:

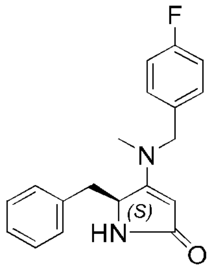

T-82

The experimental process is as follows:
The synthesis route is as follows:

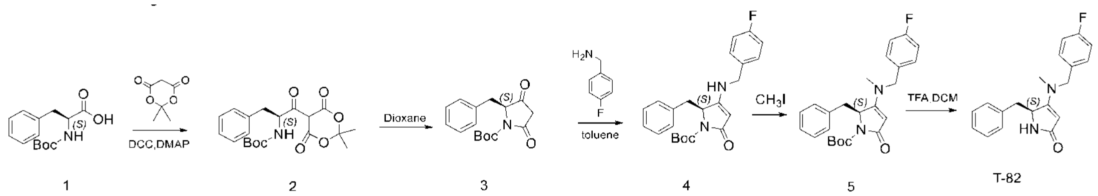


1. Synthesis of compound 4

compound 4 was obtained by referring to Example 5.

2. Synthesis of compound 5

To a 100 ml three-neck flask was added compound 4 (178 mg, 1.0 eq), displaced three times with nitrogen gas and protected with nitrogen gas, then added ultra-dry DMF to dissolve and stirred for 10 min in an ice bath, added 60% sodium hydride (36 mg, 2.0 eq) and continued stirring for 15 min, and then added methyl iodide (31ul, 1.1eq) and reacted at room temperature for 3 hours. TLC detection showed that the raw material reaction was complete. Extracted with water and ethyl acetate, the organic phase was washed with saturated sodium chloride, dried over anhydrous sodium sulfate, and purified by column chromatography to obtain 70mg of compound 5. LC-MS[M+1]: 411.2.

3. Synthesis of compound T-82

To a 100 ml three-neck flask was added compound 5 (70 mg, 1.0 eq) and DCM (2.0 ml) and stirred to dissolve, then added trifluoroacetic acid (131 ul, 10.0 eq) dropwise under an ice bath, and reacted at room temperature for 3 hours. TLC detection showed that the raw material reaction was complete. Extracted with saturated sodium bicarbonate solution and ethyl acetate, the organic phase was washed with saturated sodium chloride, dried over anhydrous sodium sulfate,

and obtained 30 mg of compound T-82 after separation and purification. HPLC purity: 95.4%. $^1$H NMR (400 MHz, Chloroform-d) δ 7.31 (q, J = 8.6, 8.0 Hz, 3H), 7.20 (dd, J = 8.6, 4.5 Hz, 4H), 7.07 (t, J = 8.6 Hz, 2H), 5.28 (s, 1H), 4.79 - 4.72 (m, 1H), 4.48 (d, J = 15.7 Hz, 1H), 4.42 (dd, J = 9.8, 3.0 Hz, 1H), 4.33 (d, J = 15.7 Hz, 1H), 3.27 (dd, J = 13.9, 3.0 Hz, 1H), 2.89 (s, 3H), 2.62 (dd, J = 13.8, 9.8 Hz, 1H).

The following compounds were synthesized by referring to the method of compound T-82:

| Compound NO. | Compound Structure | characteristic data |
|---|---|---|
| T-84 | 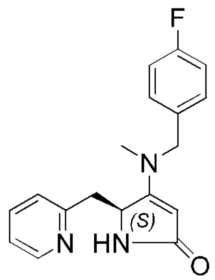 | LC-MS[M+1]: 312.1 |
| T-86 | 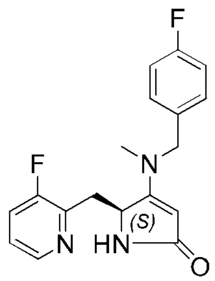 | LC-MS[M+1]: 330.1 |
| T-88 | 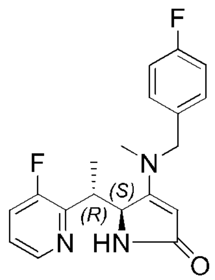 | LC-MS[M+1]: 344.1 |
| T-94 | 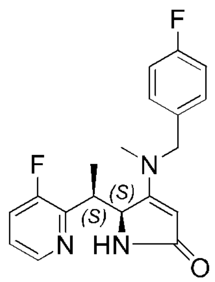 | LC-MS[M+1]: 344.1 |
| T-108 | 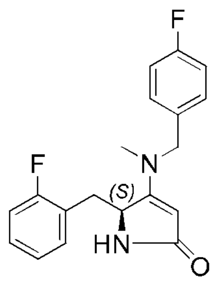 | LC-MS[M+1]: 329.1 |

(continued)

| Compound NO. | Compound Structure | characteristic data |
|---|---|---|
| T-112 | 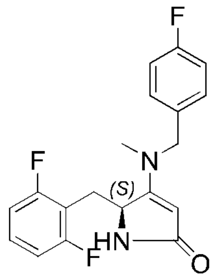 | LC-MS[M+1]: 347.1 |

**Test Example 1 Pharmacokinetic Experiment**

1. Drug Preparation

Accurately weighed approximately 10 mg of the test sample, dissolved in DMSO to a total volume of 10%, then slowly added 0.5% MC solvent to a total volume of 90% while stirring, sonicated, vortexed, and obtained a solution of visually homogeneous formulation with a concentration of 1 mg/mL; freshly prepared before use.

Pipetted 0.2 mL of the sample into a 1.5 mL centrifuge tube and stored at - 80°C for use in the concentration analysis of the drug solution.

2. Animal preparation

The animals were housed in rat cages and fasted for at least 10 hours prior to the experiment, but had access to water. On the day of the experiment, the animals were weighed and marked on the tail. Blank blood samples were collected prior to drug administration. Blood was collected from the tail vein.

3. Administration

| | |
|---|---|
| Route of administration: | gavage (p.o.) |
| Dosage concentration: | 1 mg/ml |
| Dosage: | 10 mg/kg |
| Volume: | 10 mL/kg |

Procedure: Caught the rat with left hand wearing bite-resistant gloves, hold it upright, inserted the gavage needle into its mouth and throat, and injected the drug into its stomach after confirming that there was no obvious resistance.

4. Sample collection

Test animals were sampled at 0.5 h, 1 h, 2 h, 4 h, 6 h, 8 h, 12 h, and 24 h post-administration, collected 0.1-0.2 ml of whole blood into EDTA-$Na_2$ anticoagulant tubes, inverted the tubes 3-4 times to mix thoroughly, and centrifuged at 4°C and 2000 g for 5 minutes to separate the upper layer of plasma. The plasma was promptly transferred to -80°C for storage and analysis. Blood collection was performed via the tail vein.

5. Sample Analysis and Data Processing

5.1 Sample Analysis

A quantitative detection method for the target compounds was established using Shimadzu liquid chromatography and Triple Quad TM 6500+AB mass spectrometry. The concentration of the parent drug in plasma was analyzed. The analysis results were controlled for variability using quality control samples, and the accuracy of the quality control samples should be between 80% and 120%.

5.2 Data processing

The non-compartmental model in the winnonlin Phoenix software was used to calculate the main pharmacokinetic parameters. These included the area under the pharmacokinetic curve (AUC(0-t) and AUC(0-∞)), elimination half-life ($T_{1/2}$), maximum plasma concentration ($C_{max}$), and time to reach maximum plasma concentration ($T_{max}$).
The pharmacokinetic data of the test samples obtained by the above tests are shown in Table 1.

Table 1

| Compound NO. | Tmax (h) | Cmax (ng/ml) | AUClast (h*ng/ml) |
|---|---|---|---|
| T-01 | 1.33 | 1015 | 3502 |
| T-36 | 0.42 | 1420 | 2467 |
| T-45 | 0.5 | 1877 | 3408 |
| T-47 | 1.5 | 1890 | 6730 |
| T-51 | 0.25 | 2970 | 5702 |
| T-95 | 0.83 | 1027 | 5795 |

As can be seen that compounds T-47, T-51, and T-95 have excellent pharmacokinetic properties.
Compound 50561 in WO2019029273A1 was synthesized according to WO2019029273A1, and its structural formula is as follows:

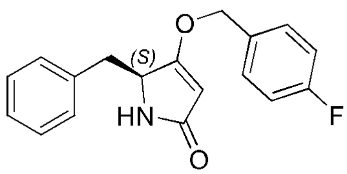

Compound 50561

The pharmacokinetic data of the test samples obtained by the above tests are shown in Table 2.

Table 2

| Compound NO. | Tmax (h) | Cmax (ng/ml) | AUClast (h*ng/ml) |
|---|---|---|---|
| Compound 50561 | 1.33 | 838 | 2499 |

As can be seen from Tables 1 and 2, the compounds of the present invention exhibit improved pharmacokinetic properties.

**Test Example 2 Brain-Penetrating Pharmacokinetic Study**

1. Drug Preparation

Solvent for Administration: 5%DMSO+10% Solutol HS15+85% physiological saline
Formulation preparation process: weighed an appropriate amount of powder, added an appropriate volume of DMSO, vortexed, then added an appropriate volume of Solutol solution, vortexed, and finally added an appropriate amount of physiological saline, vortexed and sonicated to obtain a homogeneous, clear formulation solution.

Two samples of the formulation were retained before administration, and the remaining formulation after administration was stored at 2-8°C for shipment.

2. Laboratory animals

Laboratory animals were housed in the animal facility of Suzhou Xihua New Drug Development Co., Ltd. (license number: SYXK(Su)2021-0019). The animal facility was equipped with an air conditioning system and had good ventilation. The indoor temperature was maintained 20°C-26°C, and the humidity was maintained 40%-70%. Artificial lighting was used in the animal rooms, with 12 hours of light and 12 hours of darkness (except when work lighting was required for experimental operations and cleaning). The experimental animals were allowed to eat and drink water freely.

After purchase, the animals were fed normally for at least 3 days. Rats that passed veterinary inspection and were found to be in good physical condition were included in the experiment. Each rat was marked with a tail tag. Animals in the oral administration group were fasted overnight on the day before administration and resumed feeding 4 hours after administration, and free access to water. The source and number of animals used in this experiment were shown in Table 3.

Any procedures performed on animals in the experiment must comply with the relevant SOP requirements for animal handling established by Suzhou Xihua New Drug Development Co., Ltd. and must be approved by the Institutional Animal Care and Use Committee (IACUC) of Suzhou Xihua New Drug Development Co., Ltd.

Table 3 The source and number of animals in this experiment

| Species | Strain | Source | Weight | Age (weeks) | Number of males |
|---|---|---|---|---|---|
| Rat | Sprague-Dawley (SD) | Weitong Lihua Laboratory Animal Technology Co., Ltd./Shanghai Jihui Laboratory Animal Breeding Co., Ltd. | 220 ~250 g | 6-10 | 30 |
| Note: Three animals per group. | | | | | |

3. Administration

Route of administration: gavage (p.o.)
Concentration: 1 mg/ml
Dose: 10 mg/kg
Volume: 10 mL/kg
Frequency: Single dose

Before administration, checked the condition of the drug formulation and ensured its uniformity by swirling, stirring, or shaking. Calculated the theoretical dosage volume for each SD rat in each group using the following formula.

$$\text{Theoretical dosage volume (mL)} = \frac{(\text{dose } (\text{mg} \cdot \text{kg}\hat{}(-1)\,))}{(\text{formulation concentration } (\text{mg} \cdot \text{mL}\hat{}(-1)\,))} \times \text{animal weight (kg)}$$

4. Sample collection and processing

Samples were collected from experimental rats at 0.5 h, 1 h, 2 h, 4 h, 6 h, 8 h, and 24 h after administration.

Plasma: At each time point, 0.15 mL of whole blood was collected via the jugular vein and placed in a tube containing anticoagulant (3 μL of 15% EDTA-$K_2$ solution). The tubes were stored on wet ice and centrifuged within 1 hour (2,000 g, 2-8°C, for 10 minutes) to obtain plasma. The plasma was stored in pre-chilled centrifuge tubes, rapidly frozen in dry ice, and then stored at -60°C or lower in an ultra-low temperature freezer until LC-MS/MS analysis.

Cerebrospinal fluid: the rats was euthanized using the carbon dioxide method. ~50 μL of cerebrospinal fluid was collected using a puncture method, 1 mL syringe was used with the needle bevel facing upward and the needle tip nearly horizontal to puncture the subarachnoid space, secured the needle and slowly withdrew the cerebrospinal fluid. The sample was stored in dry ice within 30 minutes after collection, then transferred it to a -90 ~ -60°C environment.

Brain tissue: A cardiac perfusion was performed first, followed by brain tissue extraction. Under deep terminal anesthesia, approximately 8 milliliters of physiological saline were used for cardiac perfusion to flush the remaining blood from the brain tissue out of the circulation. After extraction, the brain tissue was gently washed once with frozen physiological saline, blotted up the water, weighed, and then transferred to a storage environment at -90 ~ -60°C for

cryopreservation.

5. Sample Analysis and Data Processing

5.1 Sample Analysis

A quantitative detection method for the target compounds was established using Shimadzu liquid chromatography and Triple Quad TM 6500+AB mass spectrometry. The concentration of the parent drug in the sample was analyzed. The analysis results were controlled for variability using quality control samples, and the accuracy of the quality control samples should be between 80% and 120%.

5.2 Data processing

Winnonlin Phoenix 8.1.0.3530 was used to calculate pharmacokinetic parameters: $T_{max}$, $C_{max}$, AUC(0-t), AUC(0-oo), $T_{1/2}$, MRT(0-∞), etc. The pharmacokinetic data of the test samples obtained by the above tests are as shown in Table 4.

Table 4

| Compound NO. | Total drug concentration in brain tissue (h*ng/ml) | Total plasma drug concentration (h*ng/ml) | Ratio of total drug in Brain tissue/ plasma(B/P) |
|---|---|---|---|
| T-01 | 1198 | 1929 | 0.621 |
| T-47 | 11452 | 5529 | 2.07 |
| ratio of total drug in Brain tissue/ plasma(B/P)= Total drug concentration in brain tissue/ Total plasma drug concentration | | | |

It can be seen that compound T-47 has good blood-brain barrier permeability.

**Test Example 3 Electrophysiological LTP Recording**

1. Laboratory animals

| | |
|---|---|
| Strain | C57BL/6J |
| Gender | male rat |
| Age | 6-8 wks old |

2. Experimental groups

| **Group number** | **Name** | **N** |
|---|---|---|
| 1 | Control | 3 |
| 2 | T-01 | 3 |

3. Experimental Methods

3.1 Preparation of Brain Slices:

C57BL/6J mice aged 6-8 weeks was taken and anesthetized, then quickly decapitated. The scalp was cut open, and removed the skull and dura mater. Quickly removed the whole brain and placed it in 0-4°C artificial cerebrospinal fluid (ACSF) saturated with 95% $O_2$ and 5% $CO_2$ to cool it slightly. Resected the cerebellum and one-third of the forebrain,

divided the brain into two halves along the midline, and dissected the hippocampus along the cortical margin from the medial side of the brain. The brain tissue containing the hippocampus was fixed on a slide dish with glue, and cut into 400-micron-thick brain slices in the coronal plane by a vibrating microtome. The brain slices were then placed in an incubation chamber on a nylon mesh submerged beneath the liquid surface, continuously supplied with a mixed gas, and incubated at 34°C in a water bath for 0.5 h. The slices were then transferred to room temperature (26 ± 1°C) for further incubation until ready for use, with the experiment commencing 2-3 hours later.

3.2 In vitro brain slice potential recording:

Under direct visualization with a surgical microscope, a bipolar metal tungsten wire stimulation electrode with exposed tip was placed on the Schaffer collateral pathway of the CA3 region. The stimulation electrode was connected to the stimulator via an isolator, and the recording electrode was connected to a digital-to-analog converter via a microelectrode amplifier, and then connected to data acquisition software.

3.3 Electrophysiological recording:

Before recording field excitatory postsynaptic potentials (fEPSPs), the brain slice was transferred to a recording chamber, and continuously perfused the cerebrospinal fluid in a water bath with a mixture of gases (95% $O_2$, 5% $CO_2$). The stimulation electrode was inserted into the CA3 region of the hippocampus, and the recording electrode was inserted into the CA1 region of the hippocampus. When approaching the target location, the insertion depth of the stimulation electrode and recording electrode was slowly and precisely adjusted. At the same time, a pulse with a width of 1 ms was applied every 10-20 s, and the stimulus intensity was adjusted until the optimal fEPSP was achieved. Then the electrode position was fixed, and a stimulus intensity achieving 30-40% of the maximum response elicited at the optimal fEPSP was set as a stimulus intensity to record a baseline of fEPSP. The recording was continued for 20-30 min to establish a stable baseline value. Subsequently, perfusion administration was performed (initial exploratory dose of 30 μM), with the administration duration ranging from 20-30 minutes (depending on the drug's efficacy). If the drug enhanced or reduced fEPSP, normal ACSF was used for elution for 40-60 minutes to observe whether the enhanced effect could be sustained over the long term: if there was no significant change in fEPSP, the experiment was terminated. Data were recorded and amplified using an electrophysiological signal acquisition and processing system, and stored and displayed on a computer. Experimental data and images were processed using Clampfit software.

4. Data Statistics and Analysis:

All experimental data were expressed as mean ± standard error (Mean ± SEM). All statistical analyses were performed using one-way ANOVA. Significant differences were indicated as follows: $*p \leq 0.05$, $**p \leq 0.01$, $***p \leq 0.001$. $p > 0.05$ indicated no significant difference.

The electrophysiological LTP recording data of the test samples obtained by the above detection are shown in Table 5.

Table 5

| Compound NO. | fEPSP slope (% Baseline, Mean ± SEM) | | p |
|---|---|---|---|
| | Baseline | After treatment | |
| T-01 | 0.985 ± 0.014 | 1.485 ± 0.064 | 0.0038 |
| Compound 50561 | 0.969 ± 0.014 | 1.233 ± 0.040 | 0.0034 |

As shown in Table 5, compound T-01 significantly enhanced electrical signal conduction in brain slices, with $p \leq 0.05$, indicating statistically significant data. Compared with the control compound, it exhibited higher activation of LTP. Using the same detection method, compounds T-36, T-47, T-51, T-54, T-73, T-74, T-75, T-76, T-79, T-80, T-99, and T-100 of the present application all had excellent activation effects on LTP.

All literatures mentioned in the present invention are incorporated by reference herein, as though individually incorporated by reference. Additionally, it should be understood that after reading the above teaching, many variations and modifications may be made by those skilled in the art, and these equivalents also fall within the scope as defined by the appended claims.

## Claims

1. A compound, wherein the compound is a compound of formula I, or a stereoisomer, a racemate, or a pharmaceutically acceptable salt thereof,

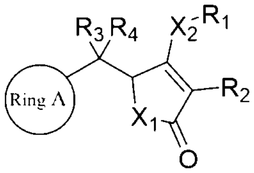

Formula I

wherein,

Ring A is selected from the group consisting of

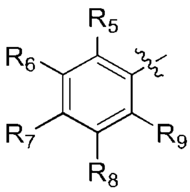
,
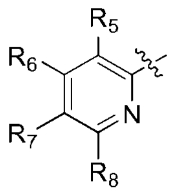
,

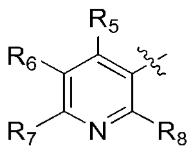
,
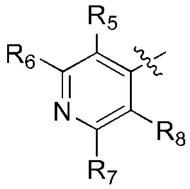
,
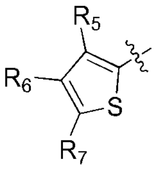
,
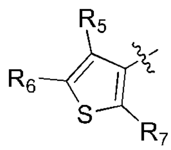
,
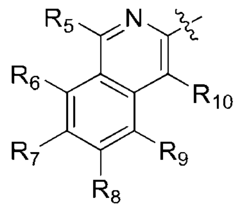
,

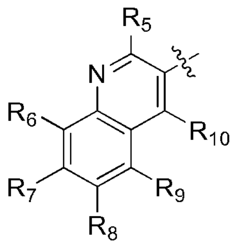
,
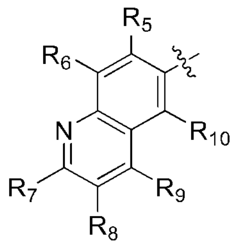
,
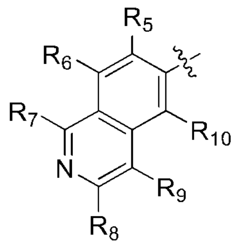
,
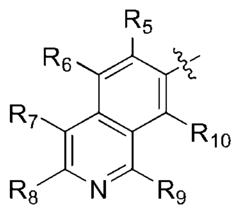
,

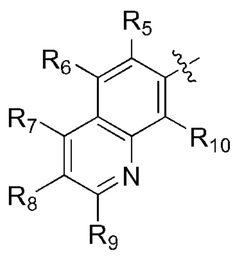
,
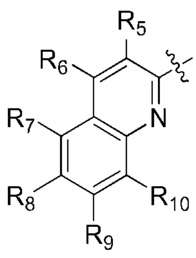
,
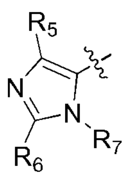
,
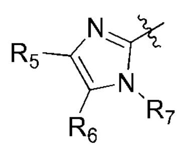
, and
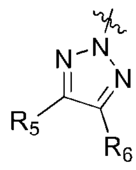
;

$X_1$ is selected from the group consisting of O, S, NH, and NR;
$X_2$ is selected from the group consisting of O, NH, and NR;
$R_1$ is selected from the group consisting of

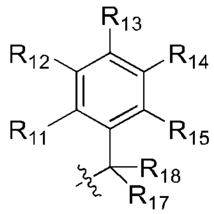
,
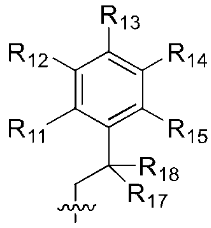
,

and

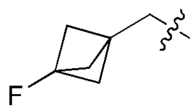
;

$R_2$ is selected from the group consisting of H, halogen, substituted or unsubstituted C1-C6 alkyl, substituted or unsubstituted C1-C6 alkoxy, and substituted or unsubstituted C3-C6 cycloalkyl;

$R_3$ and $R_4$ are independently selected from the group consisting of H, D, halogen, substituted or unsubstituted C1-C6 alkyl, substituted or unsubstituted C1-C6 alkoxy, and substituted or unsubstituted C3-C6 cycloalkyl, or $R_3$ and $R_4$ together with the carbon to which they are attached form a substituted or unsubstituted 3-7 membered cycloalkyl or a substituted or unsubstituted 3-7 membered heterocyclyl containing one or more heteroatoms selected from O, S and N;

each $R_5$, $R_6$, $R_7$, $R_8$, $R_9$, and $R_{10}$ is independently selected from the group consisting of H, D, halogen, trifluoromethyl, cyano, hydroxy, amino, substituted or unsubstituted C1-C6 alkyl, substituted or unsubstituted C1-C6 alkoxy, substituted or unsubstituted C1-C6 alkyl-$NR_{16}$-, substituted or unsubstituted C3-C6 cycloalkyl, substituted or unsubstituted C3-C6 cycloalkoxy, and substituted or unsubstituted C3-C6 cycloalkyl-$NR_{16}$-;

each $R_{11}$, $R_{12}$, $R_{13}$, $R_{14}$, and $R_{15}$ is independently selected from the group consisting of H, D, halogen, trifluoromethyl, cyano, hydroxy, amino, substituted or unsubstituted C1-C6 alkyl, substituted or unsubstituted C1-C6 alkoxy, substituted or unsubstituted C1-C6 alkyl-$NR_{16}$-, substituted or unsubstituted C3-C6 cycloalkyl, substituted or unsubstituted C3-C6 cycloalkoxy, and substituted or unsubstituted C3-C6 cycloalkyl-$NR_{16}$-;

each $R_{17}$ and $R_{18}$ is independently selected from the group consisting of H, D, C1-C6 alkyl, C1-C6 alkoxy, C3-C6 cycloalkyl and C3-C6 cycloalkoxy; or $R_{17}$ and $R_{18}$ together with the carbon to which they are attached form a substituted or unsubstituted 3-7 membered cycloalkyl or a substituted or unsubstituted 3-7 membered heterocyclyl containing one or more heteroatoms selected from O, S and N;

each R is independently a substituted or unsubstituted C1-C6 alkyl;

the "substituted" each independently refers to be substituted by one or more substituents selected from the group consisting of D, halogen, trifluoromethyl, cyano, hydroxy, amino, C1-C6 alkyl, C1-C6 alkoxy, C1-C6 alkyl-$NR_{16}$-, C3-C6 cycloalkyl, C3-C6 cycloalkoxy, and C3-C6 cycloalkyl-$NR_{16}$-;

each $R_{16}$ is independently selected from the group consisting of H and C1-C6 alkyl;

the premise is that when $X_1$ is NH, $X_2$ is O, and $R_3$, $R_4$, $R_{17}$ and $R_{18}$ are H, ring A is not

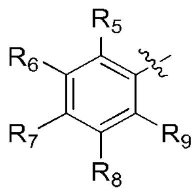
.

2. The compound according to claim 1, wherein the compound has a structure of formula II:

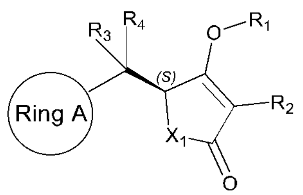
Formula II

wherein, ring A, $X_1$, $R_1$, $R_2$, $R_3$, and $R_4$ are as defined in claim 1.

**3.** The compound according to claim 1, wherein ring A is selected from the group consisting of

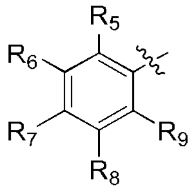 , 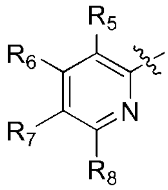 , 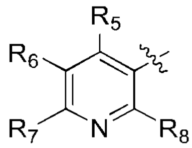 , 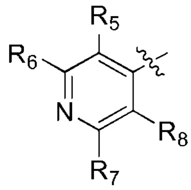 ,

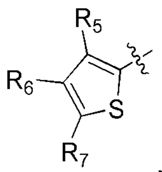 , and 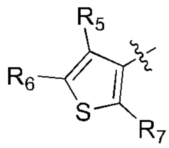 ;

$R_5$, $R_6$, $R_7$, $R_8$, and $R_9$ are as defined in claim 1.

**4.** The compound according to claim 1, wherein $R_2$ is selected from the group consisting of H, halogen, C1-C6 alkyl, C1-C6 alkoxy and C3-C6 cycloalkyl;
$R_3$ and $R_4$ are independently selected from the group consisting of H, D, halogen, C1-C6 alkyl, C1-C6 alkoxy and C3-C6 cycloalkyl.

**5.** The compound according to claim 1, wherein ring A is selected from the group consisting of

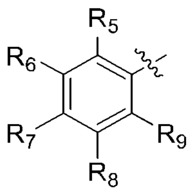 and 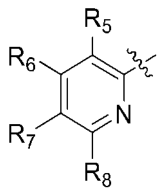 ;

$R_1$ is

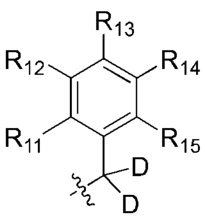 , 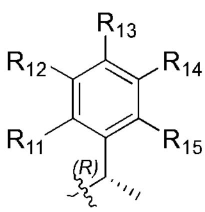 , 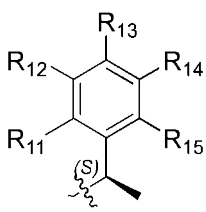 , 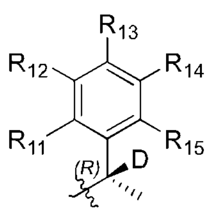 ,

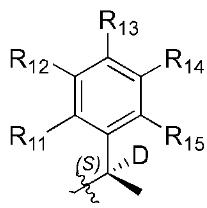 ;

each $R_5$, $R_6$, $R_7$, $R_8$, $R_9$, and $R_{10}$ is independently selected from the group consisting of H, halogen, trifluoromethyl, cyano, hydroxy, amino, C1-C6 alkyl, C1-C6 alkoxy, C3-C6 cycloalkyl and C3-C6 cycloalkoxy;
each $R_{11}$, $R_{12}$, $R_{13}$, $R_{14}$, and $R_{15}$ is independently selected from the group consisting of H, halogen, trifluoromethyl, cyano, hydroxy, amino, C1-C6 alkyl, C1-C6 alkoxy, C3-C6 cycloalkyl and C3-C6 cycloalkoxy.

**6.** The compound according to claim 1,wherein the compound is selected from the group consisting of

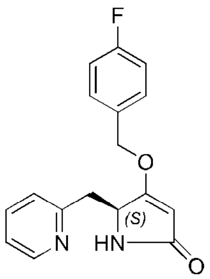

T-01

,

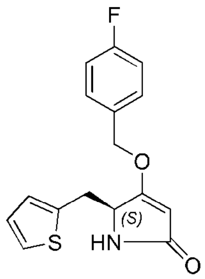

T-02

,

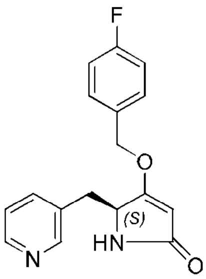

T-03

,

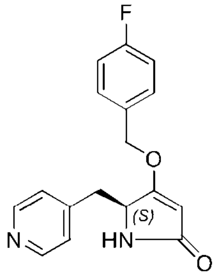

T-04

,

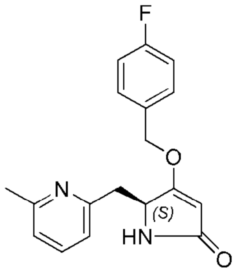

T-05

,

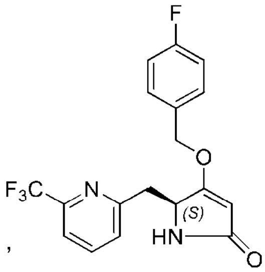

T-06

,

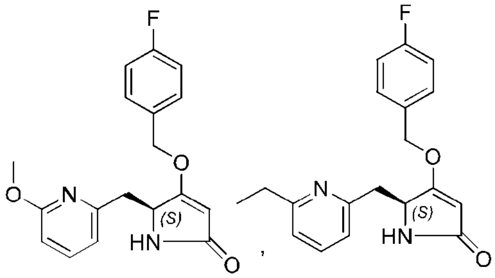

T-07

,

T-08

,

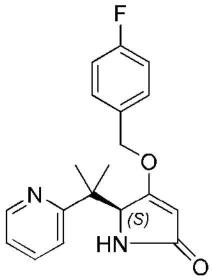

T-09

,

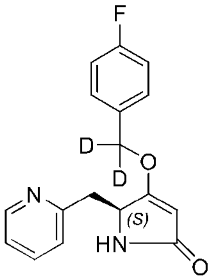

T-10

,

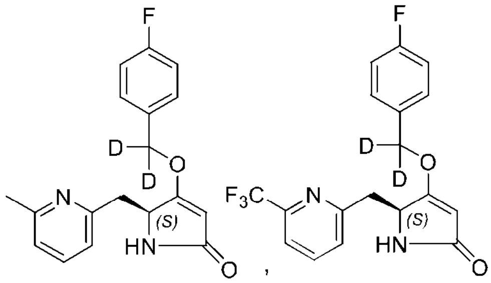

T-11

,

T-12

,

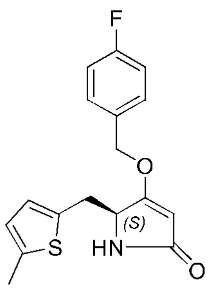

T-13

,

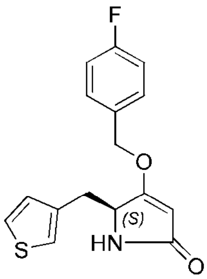

T-14

,

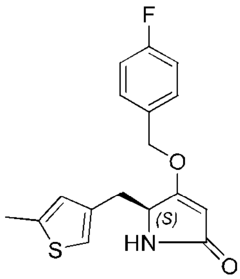

T-15

,

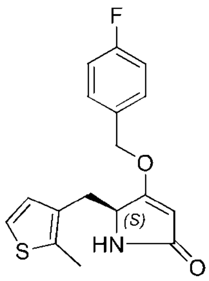

T-16

,

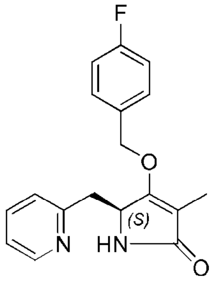 , 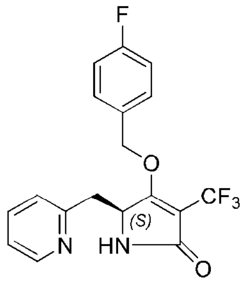 , 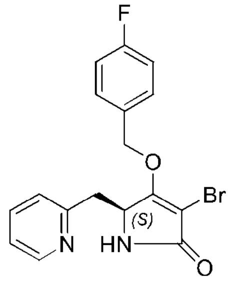 , 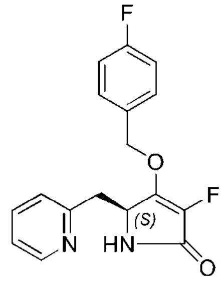 ,

T-17 T-18 T-19 T-20

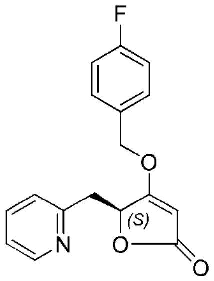 , 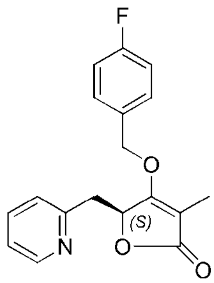 , 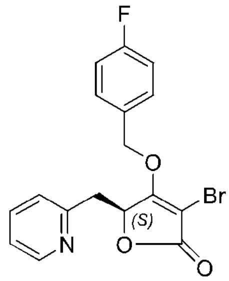 , 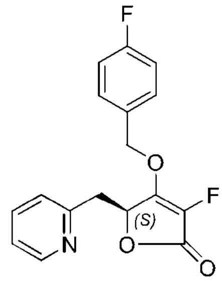 ,

T-21 T-22 T-23 T-24

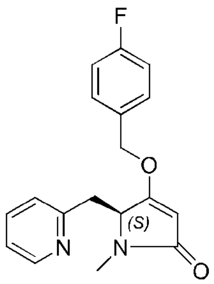 , 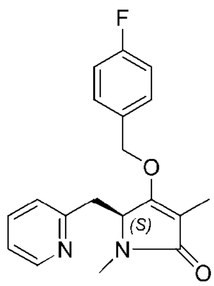 , 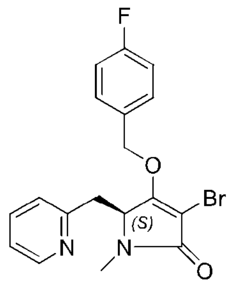 , 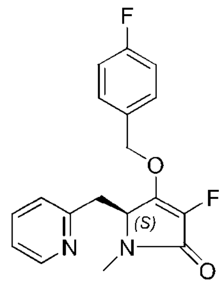 ,

T-25 T-26 T-27 T-28

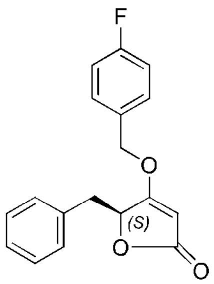 , 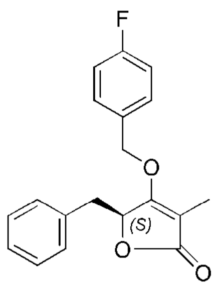 , 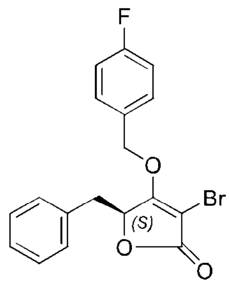 , 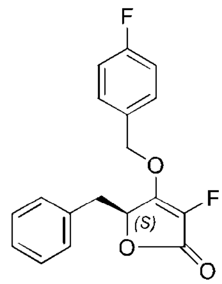 ,

T-29 T-30 T-31 T-32

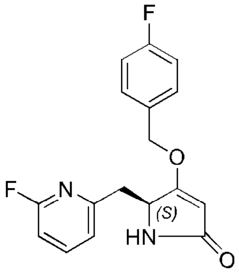
T-33
,
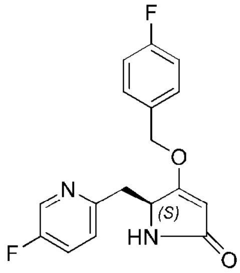
T-34
,
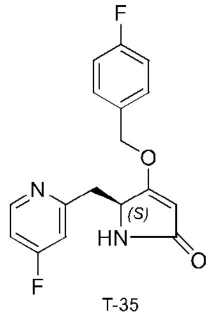
T-35
,
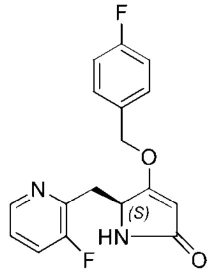
T-36
,

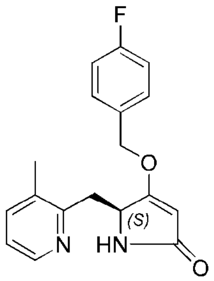
T-37
,
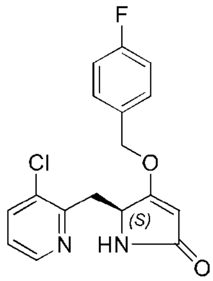
T-38
,
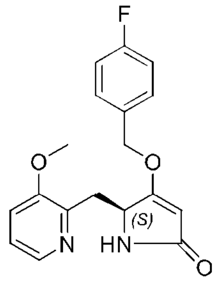
T-39
,
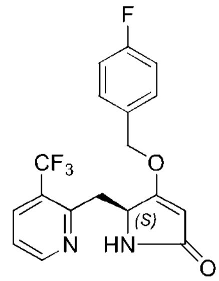
T-40
,

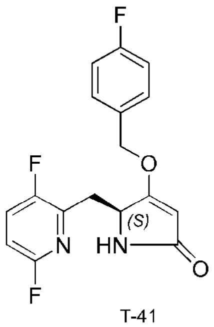
T-41
,
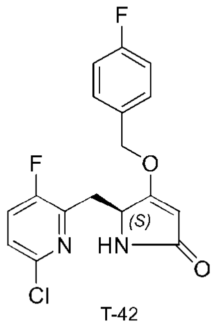
T-42
,
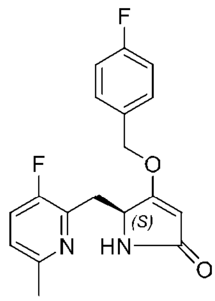
T-43
,
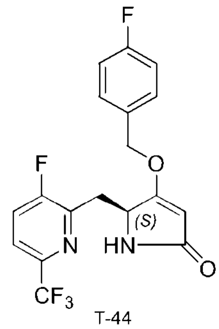
T-44
,

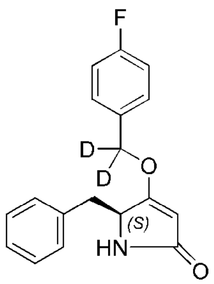
T-45
,
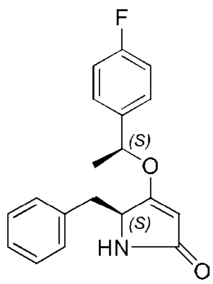
T-46
,
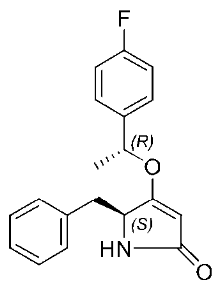
T-47
,
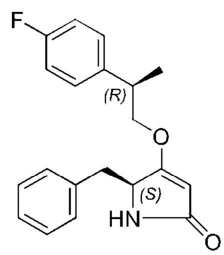
T-48
,

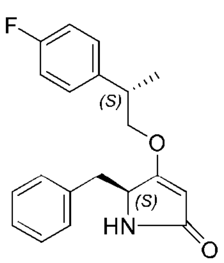
T-49
,
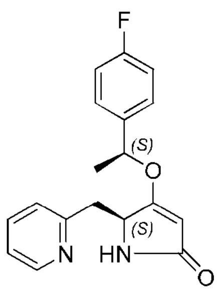
T-50
,
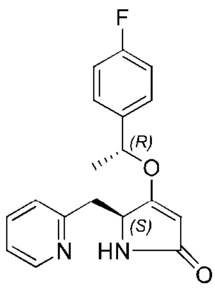
T-51
,
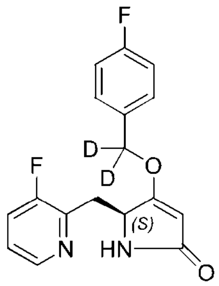
T-52
,

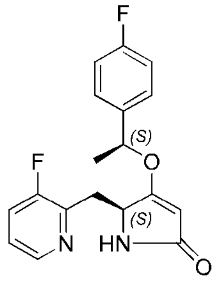
T-53
,
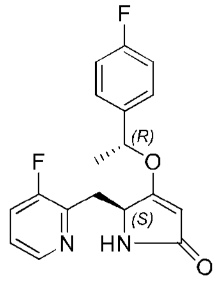
T-54
,
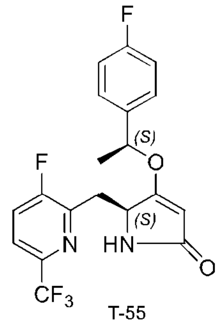
T-55
,
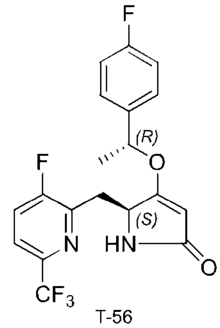
T-56
,

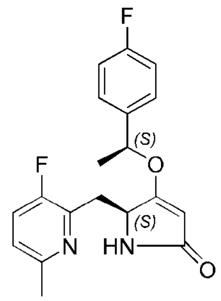
T-57
,
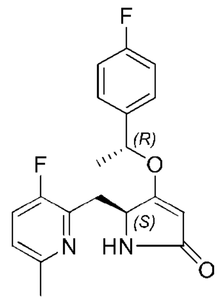
T-58
,
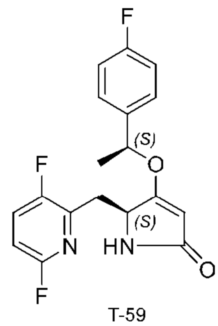
T-59
,
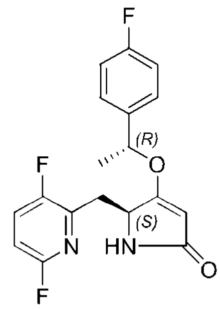
T-60
,

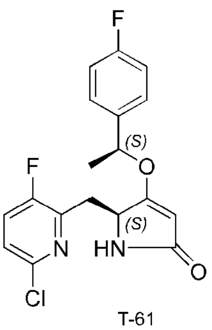
T-61
,
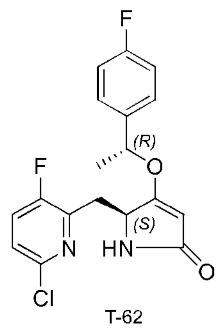
T-62
,
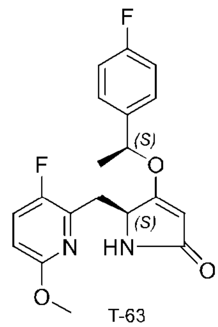
T-63
,
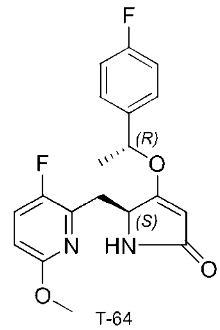
T-64
,

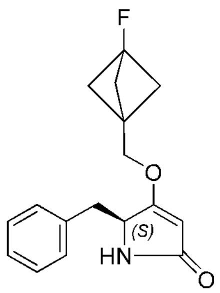
T-65
,
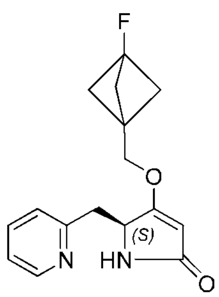
T-66
,
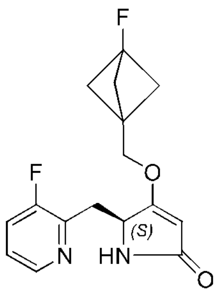
T-67
,
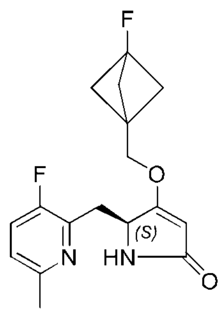
T-68
,

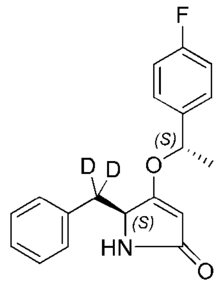 ,

T-69

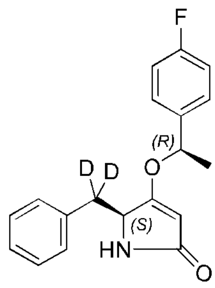 ,

T-70

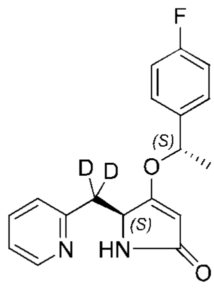 ,

T-71

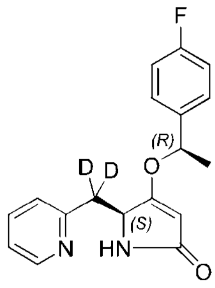 ,

T-72

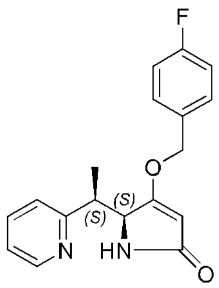 ,

T-73

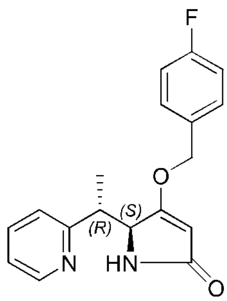 ,

T-74

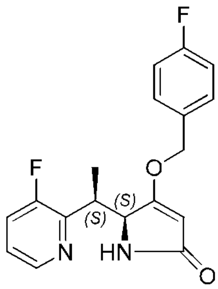 ,

T-75

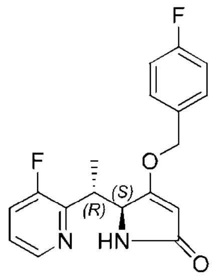 ,

T-76

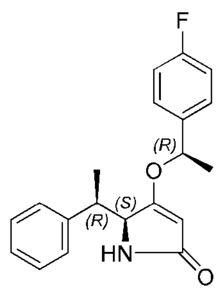 ,

T-77

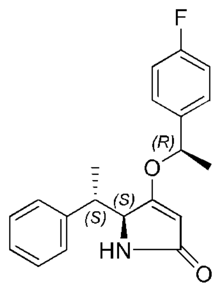 ,

T-78

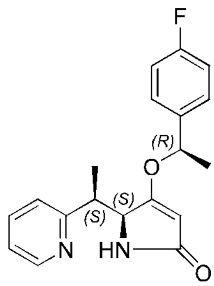 ,

T-79

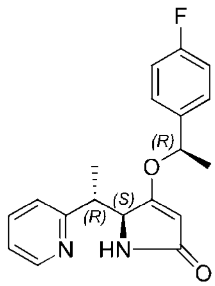 ,

T-80

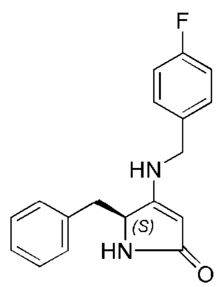 ,

T-81

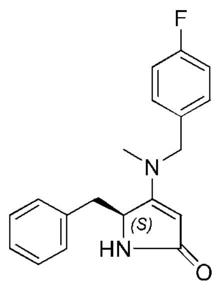 ,

T-82

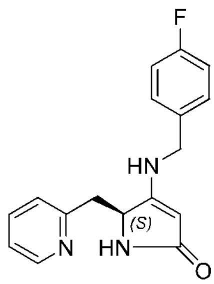 ,

T-83

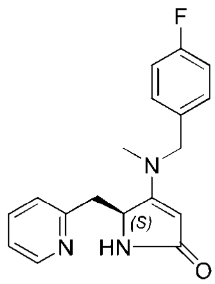 ,

T-84

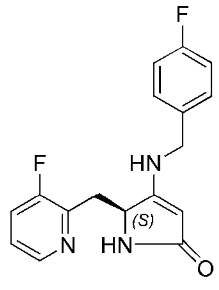 ,

T-85

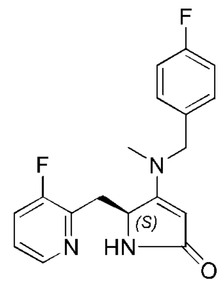 ,

T-86

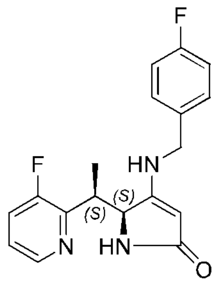 ,

T-87

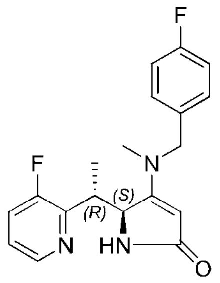 ,

T-88

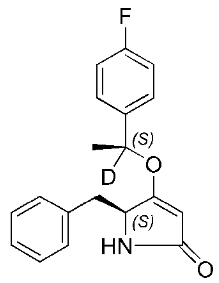 ,

T-89

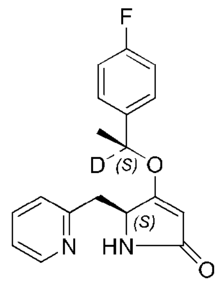 ,

T-90

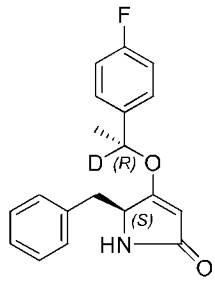 ,

T-91

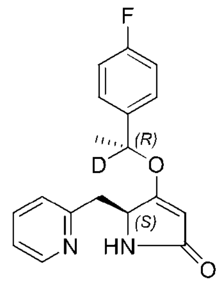 ,

T-92

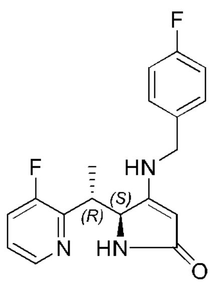 ,

T-93

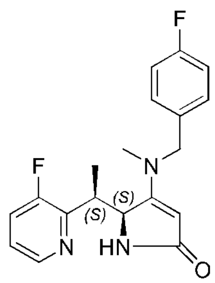 ,

T-94

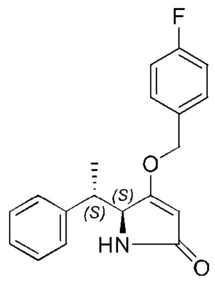 ,

T-95

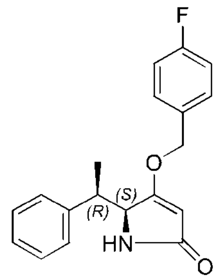 ,

T-96

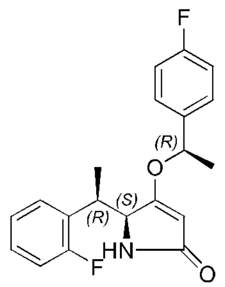 ,

T-97

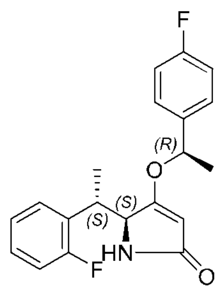 ,

T-98

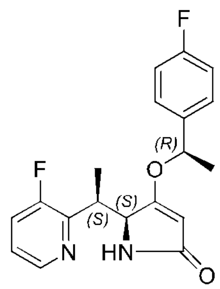 ,

T-99

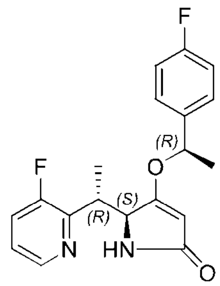 ,

T-100

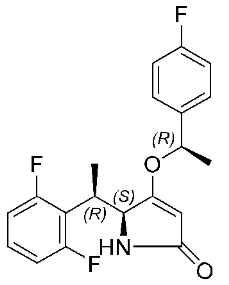 ,

T-101

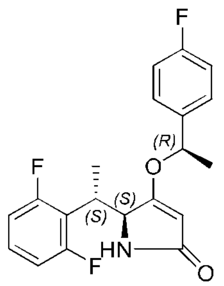 ,

T-102

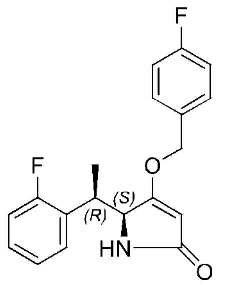 ,

T-103

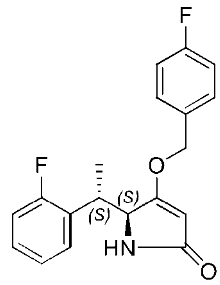 ,

T-104

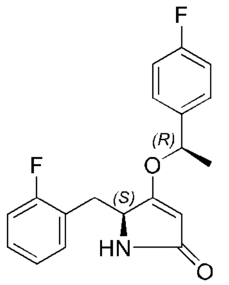 ,

T-105

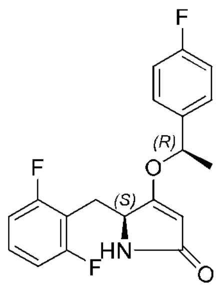 ,

T-106

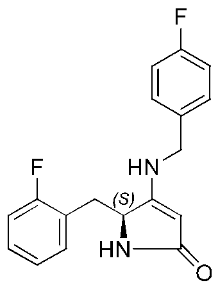 ,

T-107

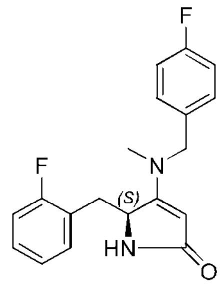 ,

T-108

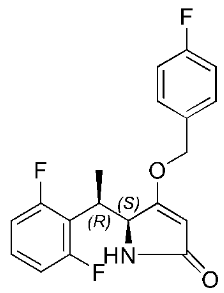 ,

T-109

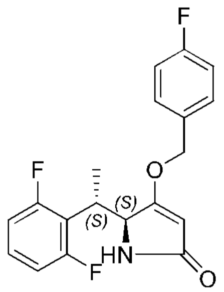 ,

T-110

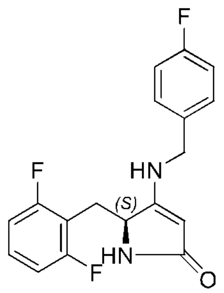 ,

T-111

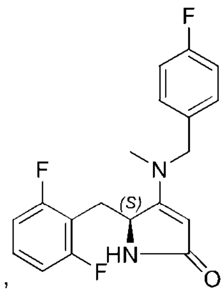 ,

T-112

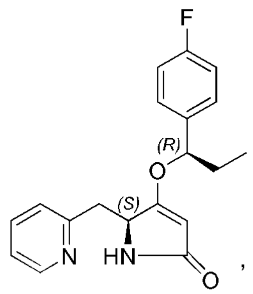 ,

T-113

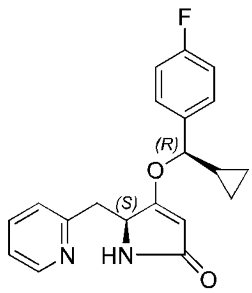 ,

T-114

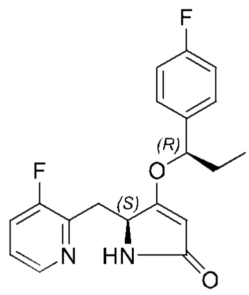 ,

T-115

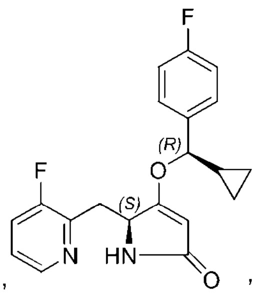 ,

T-116

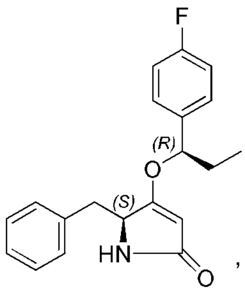,
T-117

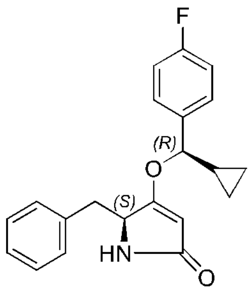,
T-118

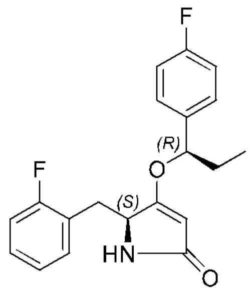,
T-119

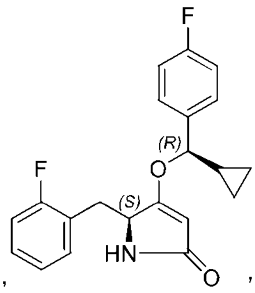,
T-120

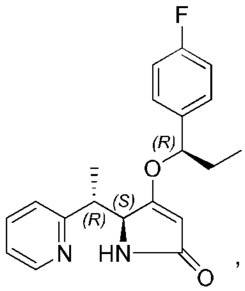,
T-121

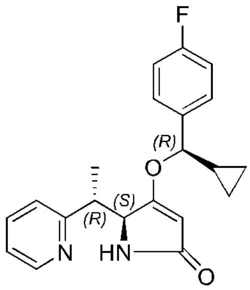,
T-122

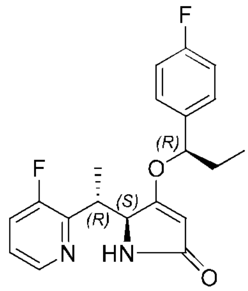,
T-123

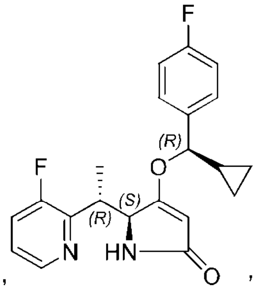,
T-124

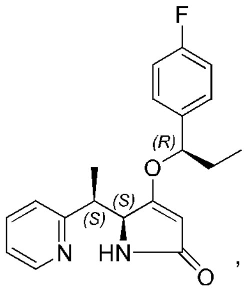,
T-125

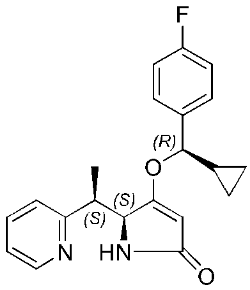,
T-126

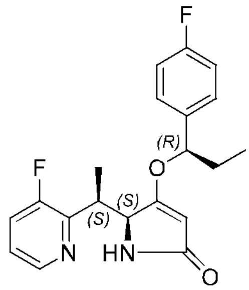,
T-127

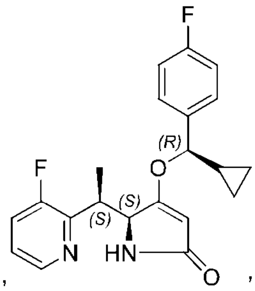,
T-128

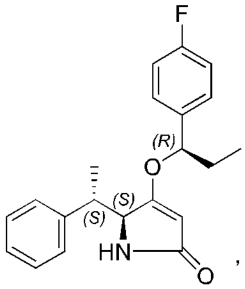,
T-129

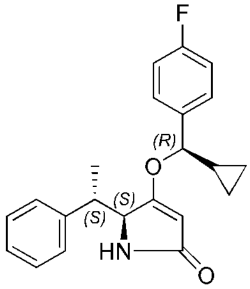,
T-130

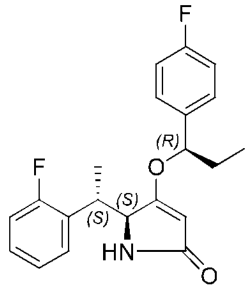,
T-131

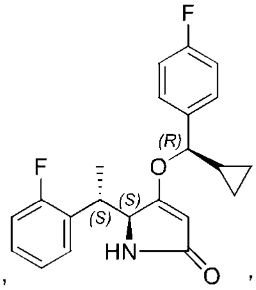,
T-132

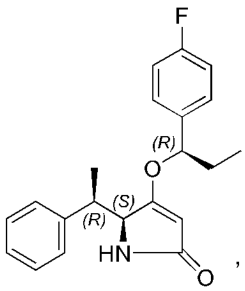
,

T-133

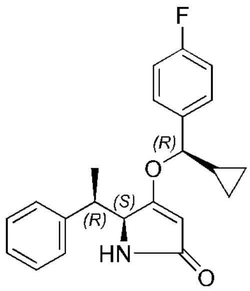
,

T-134

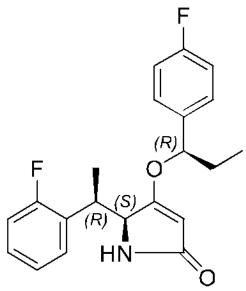
,

T-135

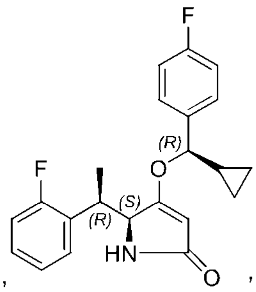
,

T-136

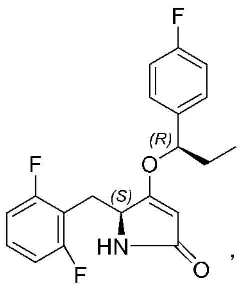
,

T-137

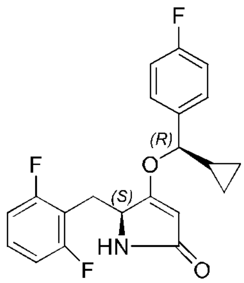
,

T-138

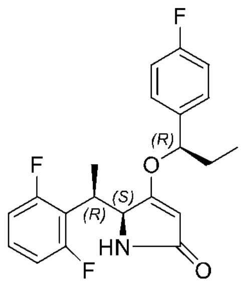
,

T-139

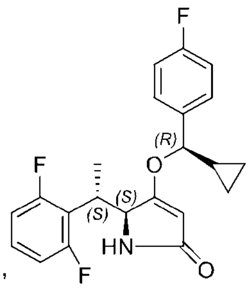

T-140

.

7. A pharmaceutical composition, comprising a pharmaceutically acceptable carrier and one or more safe and effective amounts of the compound of claim 1.

8. A use of the compound of claim 1 in the preparation of a medicament, and the medicament is used for the prevention and/or treatment of neurodegenerative diseases.

9. The use according to claim 8, wherein the neurodegenerative disease is selected from the group consisting of Alzheimer's disease, epilepsy, Parkinson's disease, Huntington's disease, amyotrophic lateral sclerosis, and spinocerebellar ataxia.

10. A use of the compound of claim 1 in the preparation of a medicament, and the medicament is used for the prevention and/or treatment of RAC1-related diseases.

**INTERNATIONAL SEARCH REPORT**

International application No.
**PCT/CN2024/070435**

**A. CLASSIFICATION OF SUBJECT MATTER**

C07D 401/06(2006.01)i; C07D 409/06(2006.01)i; C07D 405/06(2006.01)i; C07D 307/60(2006.01)i; A61K 31/365(2006.01)i; A61K 31/4439(2006.01)i; A61K 31/443(2006.01)i; A61K 31/4025(2006.01)i; A61P 25/16(2006.01)i; A61P 25/28(2006.01)i; A61P 25/08(2006.01)i; A61P 25/14(2006.01)i; A61P 25/00(2006.01)i

According to International Patent Classification (IPC) or to both national classification and IPC

**B. FIELDS SEARCHED**

Minimum documentation searched (classification system followed by classification symbols)

IPC:C07D A61K A61P

Documentation searched other than minimum documentation to the extent that such documents are included in the fields searched

Electronic data base consulted during the international search (name of data base and, where practicable, search terms used)

CNABS, CNTXT, VEN, ENTXT, ENTXTC, web of science, CNKI, 万方, WANFANG, 百度, BAIDU, caplus, marpat, registry: 同源康, 梁阿朋, 朱健, 吴像生, 陈少清, 李钧, 林嘉颖, 神经退行性, 老年痴呆, 阿尔茨海默, 阿尔兹海默, 癫痫, 帕金森, 亨廷顿, 肌萎缩性侧索硬化, 脊髓小脑性共济失调, Neurodegenerat+, Senile dementia, Alzheimer's, Epilepsy, Parkinson's, Huntington, Amyotrophic Lateral Sclerosis, Spinocerebellar Ataxia.

**C. DOCUMENTS CONSIDERED TO BE RELEVANT**

| Category* | Citation of document, with indication, where appropriate, of the relevant passages | Relevant to claim No. |
|---|---|---|
| X | Valérie Toum et al. "N-Bromosuccinimide-Promoted Cyclization of β-Carboxymethyl Enamino Esters; Synthesis of Functionalized 4-Amino-2(5H)-Furanones" *Synthesis*, No. 17, 21 July 2011 (2011-07-21), pages 2781-2788 ISSN: 0039-7881, page 2784, scheme 5 | 1, 3-5 |
| X | CN 107353239 A (JOEKAI (BEIJING) BIOTECHNOLOGY LLC.) 17 November 2017 (2017-11-17) entire document, in particular, abstract, description, paragraphs [0005]-[0027], and embodiments 1-37 | 1-10 |
| X | CN 107468690 A (JOEKAI (BEIJING) BIOTECHNOLOGY LLC.) 15 December 2017 (2017-12-15) entire document, in particular, description, paragraphs [0005]-[0028], and embodiments 1-37 | 1-10 |

☑ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **13 March 2024** | **20 March 2024** |

| Name and mailing address of the ISA/CN | Authorized officer |
|---|---|
| **China National Intellectual Property Administration (ISA/CN)** **China No. 6, Xitucheng Road, Jimenqiao, Haidian District, Beijing 100088** | Telephone No. |

Form PCT/ISA/210 (second sheet) (July 2022)

**INTERNATIONAL SEARCH REPORT**

International application No.
**PCT/CN2024/070435**

**C. DOCUMENTS CONSIDERED TO BE RELEVANT**

| Category* | Citation of document, with indication, where appropriate, of the relevant passages | Relevant to claim No. |
|---|---|---|
| A | WO 2008080988 A2 (NEUROPHARMA, S.A. et al.) 10 July 2008 (2008-07-10) entire document | 1-10 |
| A | CN 101466267 A (BAYER CROPSCIENCE AG) 24 June 2009 (2009-06-24) entire document | 1-10 |

Form PCT/ISA/210 (second sheet) (July 2022)

**INTERNATIONAL SEARCH REPORT**
**Information on patent family members**

International application No.
**PCT/CN2024/070435**

| Patent document cited in search report | Publication date (day/month/year) | Patent family member(s) | Publication date (day/month/year) |
|---|---|---|---|
| CN 107353239 A | 17 November 2017 | AU 2018314472 A1 | 13 February 2020 |
| | | AU 2018314472 B2 | 27 August 2020 |
| | | AU 2018314472 B9 | 17 September 2020 |
| | | WO 2019029274 A1 | 14 February 2019 |
| | | US 2020181081 A1 | 11 June 2020 |
| | | US 11332440 B2 | 17 May 2022 |
| | | ES 2920132 T3 | 01 August 2022 |
| | | EP 3647306 A1 | 06 May 2020 |
| | | EP 3647306 A4 | 07 April 2021 |
| | | EP 3647306 B1 | 06 April 2022 |
| | | CN 107353239 B | 18 June 2019 |
| CN 107468690 A | 15 December 2017 | EP 3643311 A1 | 29 April 2020 |
| | | EP 3643311 A4 | 14 April 2021 |
| | | WO 2019029273 A1 | 14 February 2019 |
| | | AU 2018314471 A1 | 13 February 2020 |
| | | AU 2018314471 B2 | 27 May 2021 |
| | | US 2020361868 A1 | 19 November 2020 |
| | | US 11130733 B2 | 28 September 2021 |
| | | CN 107468690 B | 31 January 2020 |
| WO 2008080988 A2 | 10 July 2008 | EP 1939192 A1 | 02 July 2008 |
| CN 101466267 A | 24 June 2009 | JP 2009539900 A | 19 November 2009 |
| | | US 2010056620 A1 | 04 March 2010 |
| | | KR 20090020699 A | 26 February 2009 |
| | | TW 200814931 A | 01 April 2008 |
| | | EP 2034843 A1 | 18 March 2009 |
| | | MX 2008015840 A | 09 January 2009 |
| | | DE 102006027730 A1 | 20 December 2007 |
| | | WO 2007144089 A1 | 21 December 2007 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2019029273 A1


### Non-patent literature cited in the description


- *Human Molecular Genetics*, 2020, vol. 29 (5)
- *PNAS*, 2007, vol. 104 (2)
- **SAMBROOK et al.** Molecular Cloning: Laboratory Manual. Cold Spring Harbor Laboratory Press, 1989